# EUROPEAN PATENT SPECIFICATION

(11) **EP 0 981 362 B1**
(45) Date of publication and mention of the grant of the patent: **05.11.2003**
(21) Application number: 98922546.1
(22) Date of filing: 15.05.1998
(51) Int. Cl.: A61K 38/26, A61K 38/30, A61K 38/27, A61K 35/38, G01N 33/50, C12N 5/06, C12N 5/08

(54) **USE OF GLP-2 AGONISTS ENHANCING FUNCTIONING OF THE UPPER GASTROINTESTINAL TRACT**
VERWENDUNG VON GLP-2 AGONISTEN ZUR VERBESSERUNG DER WIRKUNG DES OBEREN GASTROINTESTINALTRAKTES
UTILISATION D'AGONISTES DU GLP-2 POUR AMELIORER LE FONCTIONNEMENT DU TRACTUS GASTRO-INTESTINAL SUPERIEUR

(30) Priority: 16.05.1997 US 46754 P; 23.07.1997 GB 9715481; 13.04.1998 US 59504
(43) Date of publication of application: 01.03.2000
(73) Proprietor: 1149336 ONTARIO INC., Toronto, Ontario M6B 3G3 (CA)
(72) Inventor: DRUCKER, Daniel, J., Toronto, Ontario M6B 3G3 (CA)
(74) Representative: Horner, Martin Grenville
(86) International application number: CA9800497
(87) International publication number: WO98052600

(56) References cited:
- WO-A-96/32414
- WO-A-97/39031
- WO-A-98/24813
- WO-A-98/25644
- D.J. DRUCKER ET AL.: "INDUCTION OF INTESTINAL EPITHELIAL PROLIFERATION BY GLUCAGON-LIKE PEPTIDE 2." PROCEEDINGS OF THE NATIONAL ACADEMY OF SCIENCES OF USA, vol. 93, July 1996, pages 7911-7916, XP000602070 WASHINGTON US cited in the application
- D.J. DRUCKER ET AL.: "INTESTINAL RESPONSE TO GROWTH FACTORS ADMINISTERED ALONE OR IN COMBINATION WITH HUMN [GLY2]GLUCAGON-LIKE PEPTIDE 2." AMERICAN JOURNAL OF PHYSIOLOGY: GASTROINTESTINAL AND LIVER PHYSIOLOGY, vol. 273, no. 6 PART 1, December 1997, pages G1252-G1262, XP002060300 WASHINGTON, DC, US

## Description

### Field of Invention

This invention relates to glucagon-related peptides and their use, either alone or in combination with other peptide hormones, for the prevention or treatment of disorders involving the upper gastrointestinal tract.

### Background of the Invention

Glucagon-like peptide-2 (GLP-2) is a 33 amino acid peptide expressed in a tissue-specific manner from the pleiotropic glucagon gene. GLP-2 shows remarkable homology in terms of amino acid sequence to glucagon and Glucagon-Like Peptide-1 (GLP-1). Further, different mammalian forms of GLP-2 are highly conserved. For example, the human GLP-2 and degu (a south American rodent) GLP-2 differ from rat GLP-2 by one and three amino acids respectively. When given exogenously, GLP-2 can produce a marked increase in the proliferation of small intestinal epithelium of test mice, apparently with no undesirable side effects (Drucker et al., 1996, *PNAS:USA* 93:7911-7916). Subsequently it was shown that peptide analogs of native GLP-2 with certain modifications to the peptide sequence possess enhanced trophic activity at the small intestine (see co-pending application U.S. Serial No. 08/669,791, filed June 28, 1996, incorporated herein by reference). It has further been demonstrated that GLP-2 can proliferate the tissue of the large intestine (co-pending applications U.S. Serial No. 08/763,177, filed December 10, 1996, and U.S. Serial No. 08/850,664, filed on May 2, 1997, and Litvak et al., 1997, *Gastroenterology,* vol. 112 (4 Suppl.), page A1455, all of which are incorporated herein by reference). Moreover, GLP-2 has also been shown to increase D-glucose maximal transport rate across the intestinal basolateral membrane (Cheeseman and Tseng, 1996, *American Journal of Physiology* 271:G477-G482).

A number of peptide hormones, structurally unrelated to GLP-2, have been demonstrated to have varying degrees of trophic activity. For example, Insulin-Like Growth Factor-2 (IGF-2) has been shown to promote mitosis of the crypt cells of the small intestine *in vivo* (U.S. Patent No. 5,482,926). Insulin-Like Growth Factor-1 (IGF-1), which shares 64% sequence identity with IGF-2, and peptide analogs thereof have also been shown to increase the growth of gut tissue *in vivo* (WO 91/12018). Growth Hormone (GH) has been shown to have a number of physiological effects, including increasing proliferation of the intestinal mucosa (*see*, for example, Willmore, U.S. Patent No. 5,288,703), thereby enhancing the absorptive capacity of the gut. However, none of the above peptide hormones possess the efficacy or specificity of GLP-2 in promoting proliferation of the tissue of the lower gastrointestinal tract.

### Summary of the Invention

The invention is based, in part, on the discovery that GLP-2 receptor agonists act to enhance functioning of the upper gastrointestinal tract. Specifically, it has been demonstrated that GLP-2 can proliferate the tissue of the esophagus and stomach. It is accordingly a general object of the present invention to exploit GLP-2 receptor agonists for therapeutic and related purposes.

In particular, it has been demonstrated that GLP-2 and peptidic analogs of GLP-2 can cause proliferation of the tissue of upper gastrointestinal tract. Thus, one aspect the invention provides a method of proliferating the tissue of the upper gastrointestinal tract in a subject in need thereof comprising delivering to the upper gastrointestinal tract of the subject an upper gastrointestinal tract proliferating amount of GLP-2 or a GLP-2 analog.

In addition, it has been demonstrated that GLP-2 can ameliorate nonsteroidal anti-inflammatory drug (NSAID) induced gastrointestinal toxicity. Thus, the invention provides methods of therapeutically or prophylactically treating a subject with or at risk of an inflammatory condition of the gastrointestine involving the upper gastrointestinal tract, comprising delivering to the upper gastrointestinal tract an effective amount of GLP-2 or a GLP-2 analog.

More particularly, and according to one aspect of the invention, there is provided a method of treating a subject suffering from a condition involving the upper gastrointestinal tract, wherein GLP-2 or a GLP-2 analog is delivered to the upper gastrointestinal tract in an amount capable of ameliorating the condition.

In a related aspect of the invention, there is provided a method of treating a subject having a damaged, partially resected, eroded or inflamed esophagus comprising the step of delivering to the subject a upper gastrointestinal tract damage or inflammation ameliorating amount of GLP-2 or an analog of GLP-2 in a pharmaceutically or veterinarily acceptable carrier. In a further aspect, GLP-2 or a GLP-2 analog is provided in a pharmaceutically or veterinarily acceptable form in an amount effective to cause proliferation of the upper gastrointestinal tract.

In a further aspect of the invention, there is provided a method of treating a subject having a damaged, atrophic or inflamed stomach comprising the step of delivering to the subject a stomach damage or inflammation ameliorating amount of GLP-2 or an analog of GLP-2 in a pharmaceutically or veterinarily acceptable carrier. In a further aspect, GLP-2 is provided in a pharmaceutically or veterinarily acceptable form in an amount effective to cause proliferation of the tissue of the stomach.

In another aspect, the invention provides a method of prophylactically treating a subject at risk of developing an inflammatory condition of the gastrointestine involving the upper gastrointestinal tract comprising the steps of:
a) identifying a subject at risk of developing an inflammatory condition involving the upper gastrointestinal tract; and
b) administering to the subject an amount of GLP-2 or a GLP-2 analog effective to inhibit onset of the inflammatory condition.

In another aspect of the invention, there is provided a method to identify peptides useful to treat inflammatory conditions involving the upper gastrointestinal tract comprising the steps of:
a) obtaining an analog of a vertebrate GLP-2 peptide, the analog having at least one amino acid substitution, deletion, addition, or an amino acid with a blocking group;
b) inducing an inflammatory condition of the upper gastrointestinal tract in a test animal;
c) treating the test animal having an induced inflammatory condition of the upper gastrointestinal tract with the analog using a regimen capable of eliciting an amelioration of the inflammatory condition of the upper gastrointestinal tract when utilized for human [Gly²]GLP-2; and
d) determining the effect of the analog on the health status or mortality of the test animal compared with control animals not receiving the peptide or determining the mass of the upper gastrointestinal tract of test animals compared to control animals not receiving peptide.

In another aspect of the invention, there is provided a method to identify peptides useful to prevent or ameliorate inflammatory conditions involving the upper gastrointestinal tract comprising the steps of:
a) obtaining an analog of a vertebrate GLP-2 peptide, the analog having at least one amino acid substitution, deletion, addition, or an amino acid with a blocking group;
b) treating a test animal with the analog using a regimen capable of eliciting an amelioration of the inflammatory condition of the upper gastrointestinal tract when utilized for human [Gly²]GLP-2;
c) inducing an inflammatory condition of the upper gastrointestinal tract in a test animal;
e) determining the effect of the analog on the health status or mortality of the test animal compared with control animals not receiving the peptide or determining the mass of the upper gastrointestinal tract of test animals compared to control animals not receiving peptide.

In another aspect, the invention provides a method of prophylactically treating a subject at risk of developing an inflammatory condition of the gastrointestine involving the upper gastrointestinal tract comprising the steps of:
a) identifying a subject at risk of developing an inflammatory condition involving the upper gastrointestinal tract; and
b) administering to the subject an amount of GLP-2 or a GLP-2 analog effective to inhibit onset and/or ameliorate the development of the inflammatory condition.

In a related aspect of the invention, there is provided a method useful to identify peptides capable of proliferating the tissue of the upper gastrointestinal tract comprising the steps of:
a) obtaining an analog of a vertebrate GLP-2 peptide, the analog having at least one amino acid substitution, deletion, addition, or an amino acid with a blocking group;
b) delivering the analog to the upper gastrointestinal tract of the test animal using a regimen capable of proliferating the upper gastrointestinal tract when utilized for human [Gly²]GLP-2; and
c) assessing the increase in the mass, length, or total protein content of the upper gastrointestinal tract or a representative portion after completion of the treatment regime.

In another aspect, the invention provides a method for growing upper gastrointestinal tract tissue or cells therefrom, which comprises the step of culturing the tissue or cells in a culturing medium supplemented with a growth promoting amount of GLP-2 or a GLP-2 analog.

The invention is also based, in part, on the expectation that GLP-2 or a GLP-2 analog will act synergistically with the peptide hormones IGF-1 and/or GH to promote the proliferation of cells. Furthermore, coadministration of GLP-2 with either IGF-1 or GH results in cell proliferation. The trophic effects on the small and large intestines of this combination therapy are greater than that seen with any one of GLP-2, IGF-1 or GH alone. An additional aspect of the invention is the coadministration of GLP-2 with IGF-2 or GH to promote growth.

Therefore, one aspect of the invention is a composition for promoting the growth of upper gastrointestinal tract tissue in a mammal which comprises GLP-2, or a trophic analog of GLP-2, in admixture with at least one other peptide hormone selected from the group consisting of IGF-1, IGF-2, and GH, and a pharmaceutically acceptable carrier. These compositions of the invention may alternatively include analogs of GLP-2, IGF-1, IGF-2 and/or GH which exhibit trophic activities.

In another aspect, the invention provides a method of promoting the growth of upper gastrointestinal tract tissue in a mammal, comprising treating the mammal to elevate serum levels of GLP-2, or an analog of GLP-2, and at least one other peptide hormone selected from the group consisting of IGF-1, analogs of IGF-1, IGF-2, analogs of IGF-2, GH, and analogs of GH. Such methods of the invention include co-administering to a mammal an effective amount of GLP-2 and an effective amount of at least one other peptide hormone selected from the group consisting of IGF-1, analogs of IGF-1, IGF-2, analogs of IGF-2, GH, and analogs of GH, treating the mammal with hormones or small organic molecules which act to elevate serum levels of GLP-2, IGF-1, IGF-2, and/or GH, and performing gene therapy to induce cells in the mammal to endogenously produce GLP-2, IGF-1, IGF-2, and/or GH or to engineer cells to produce GLP-2, IGF-1, IGF-2, or GH, alone or in combination, that may then be implanted in a mammal to produce the desired biological effect.

Also provided by the invention is a method for treating a subject to enhance functioning of the upper gastrointestinal tract, comprising treating the mammal to elevate serum levels of GLP-2, or an analog of GLP-2, and at least one other peptide hormone selected from the group consisting of IGF-1, analogs of IGF-1, IGF-2, analogs of IGF-2, GH, and analogs of GH.

The compositions and methods of the invention are useful for restoring or maintaining gastrointestinal function, for promoting the healing and regrowth of injured or ulcerated/inflamed intestinal mucosa, for reducing the risk of enteric disease, for enhancing the nutritional status of a mammal, and for the treatment or prevention of nutritional or upper gastrointestinal disorders in a mammal, particularly a human.

Alternatively, the compositions and methods of the invention can be used to promote proliferation of the upper gastrointestinal tract in a healthy mammal, *e.g*., to enable increased absorption of nutrients in cattle allowing earlier weaning or increased milk and meat production.

In yet another aspect of the invention, there is provided a use of GLP-2, or analogs of GLP-2, for the manufacture of a pharmaceutical or veterinary preparation for the enhancement of upper gastrointestinal tract tissue growth. In a particularly preferred aspect of the invention, such preparations also include another peptide hormone selected from the group consisting of IGF-1, analogs of IGF-1, IGF-2, analogs of IGF-2, GH, and analogs of GH.

Still further, the invention provides kits comprising GLP-2, or analogs of GLP-2, and at least one other peptide hormone selected from the group consisting of IGF-1, analogs of IGF-1, IGF-2, analogs of IGF-2, GH, and analogs of GH. Such compositions are provided in a therapeutically effective unit dose or multi-dose amount.

Also provided by the instant invention is a method for promoting the growth of upper gastrointestinal tract tissue or cells which comprises the step of culturing said tissue or cells in a culturing medium containing a growth promoting combination of both GLP-2, or a GLP-2 analog, and at least one other peptide hormone selected from the group consisting of IGF-1, analogs of IGF-1, IGF-2, analogs of IGF-2, GH, and analogs of GH. These methods may be performed on cells in culture and *in vivo*.

In yet another aspect of the invention, there is provided a method in which treatment of a patient to restore upper gastrointestinal tract tissue is performed by the steps of: (a) culturing tissue or cells derived from the patient with a tissue growth promoting amount of a combination of GLP-2 or an GLP-2 analog, and at least one other peptide hormone selected from the group consisting of IGF-1, analogs of IGF-1, IGF-2, analogs of IGF-2, GH, and analogs of GH, and then (b) implanting said tissue or cells in the patient to be treated.

Finally, the invention also provides a method for determining the activity of a hormone when used in combination with GLP-2 which comprises the steps of: (1) coadministering the hormone with an amount of GLP-2, or a GLP-2 analog, to a test mammal; (2) assessing the subsequent growth of upper gastrointestinal tract tissue in the test mammal; and (3) determining whether the growth of upper gastrointestinal tract tissue in the test mammal is enhanced relative to control mammals treated with GLP-2 alone.

### Brief Description of the Figures

Figure 1 illustrates the change in the number of cells which stain with bromodeoxyuridine, in the mucosal epithelium and muscle layer of the esophagus, after treatment with GLP-2.
Figure 2 illustrates the change in total protein content of the esophagus after treatment with GLP-2.
Figure 3 illustrates the change in weight of the stomach after treatment with GLP-2.

### Detailed Description of the Invention

The invention relates to therapeutic, prophylactic, and related uses of GLP-2 and GLP-2 analogs, in particular for the amelioration of medical or veterinary conditions in which functioning of the upper gastrointestinal tract is impaired by disease or injury. For example, the method is usefully applied to treat subjects suffering from an inflammatory condition involving the upper gastrointestinal tract, subjects who have undergone resection of the upper gastrointestinal tract, or subjects whose upper gastrointestinal tract has been damaged by toxins and the like.

As used herein the term "upper gastrointestinal tract" means that section of the gastrointestinal (GI) tract which comprises the esophagus and the stomach. The esophagus is that section of the GI tract that arises proximally at the pharyngoesophageal junction and continues distally through the posterior mediastinum to end at the gastroesophageal junction. The stomach is a distensible structure extending from the gastroesophageal junction to the duodenum.

As used herein the term "subject" includes a human or other mammal and including livestock and pets.

As used herein the term "GLP-2 receptor agonist" means any molecule which on binding to the GLP-2 receptor results in activation of the GLP-2 receptor, and includes for example GLP-2 or peptidic analogs of GLP-2. Recently it has been demonstrated that the GLP-2 receptor is a G-protein coupled receptor. Nucleic acid encoding the GLP-2 receptor has been isolated (see co-pending applications U.S. Serial No.08/767,224,filed December 13, 1996, corresponding to WO 98/25955, and U.S. Serial No.08/845,546, filed April 24, 1997 corresponding to EP97951013.8. Thus, methods commonly used in this field to identify G-protein coupled receptor agonists may be usefully applied to the GLP-2 receptor. One particularly useful methodology for assessing compounds for GLP-2 receptor agonist activity is disclosed in the abovementioned co-pending applications. Briefly, suitable cells such as COS cells are transformed with GLP-2 receptor encoding nucleic acid such that functional receptor is provided at the cell surface. Thereafter agonist activity of a test compounds can be assessed by contacting transformed cells by the test compound; an increase in the intracellular level of cyclic adenosine monophosphate in response to binding of the test compound to the transformed cells indicates agonist activity.

GLP-2 peptide analogs and selected chemical libraries, may be screened for GLP-2 receptor agonist activity using this approach. Guidance on the types of peptidic analogs that may be usefully employed in this method is given herein and in co-pending applications U.S. Serial Nos. 08/632,533, corresponding to EP96908973.9, and 08/631,273, corresponding to EP97916280.7, both filed on April 12, 1996. Moreover, any of the commercially available chemical libraries may be usefully screened for small molecule GLP-2 receptor agonists using high throughput or ultra high throughput screening technology. Peptidic analogs of GLP-2 and small molecule agonists identified as GLP-2 receptor agonists may be screened for therapeutic and related utility to treat conditions involving the upper gastrointestinal tract using the models described herein and in the scientific literature.

Any subject requiring enhancement of the activity of the upper gastrointestinal tract may potentially be a candidate for treatment with a GLP-2 agonist according to the invention. In particular, one group of conditions that may be beneficially treated according to the invention are diseases involving the esophagus. Human patients are typically diagnosed as having such a condition after manifesting one or more of the following symptoms: pain or discomfort on swallowing, pain or discomfort on swallowing liquids or solids, heartburn, a bitter taste in the mouth, a sensation of food sticking in the throat or during swallowing (often manifest as a lump in the throat) and shortness of breath. Visualization of the esophagus using esophagogastroscopy can be used to confirm the presence of damage or disease of the esophagus. Alternatively, the esophagus can be visualized radiologically using a barium contrast swallowing X-ray, or CT scan with an oral contrast material. The function of the esophagus can also be assessed by manometry. The presence of esophageal disease, particularly esophagitis, can be assessed functionally using the Bernstein test. For a more precise diagnosis of esophageal disease, biopsies can be taken of the esophageal mucosa for analysis of histological evidence of inflammation, and further, cultures can be made of this material to determine the presence of bacterial, viral or fungal infection and inflammation.

Inflammatory conditions of the esophagus include: inflammation-vasculitis, post-infection inflammation, infiltrative disorder, *e.g*., scleroderma and amyloid, as well as conditions wherein the inflammation results from the action of a toxin such as alcohol, acid, and alkali, such as from an accidental overdose.

Another group of conditions that may be beneficially treated, according to the invention, are diseases involving the stomach, *e.g.,* peptidic ulcer. Human patients are typically diagnosed as having stomach disorders after manifesting symptoms of abdominal pain, either when at rest or with eating or both. However, symptoms of stomach disorder can be non-specific and it can be difficult to differentiate between pain from the esophagus and the stomach.

Additionally, GLP-2 may be beneficially administered to patients who can be demonstrated to be at risk of developing a malfunctioning of the upper gastrointestinal tract to protect against onset of the condition or reduce the severity of the attack. Such patients include smokers and patients with blood group O which is associated with duodenal ulcer and prepyloric ulcer, patients receiving or about to receive one or more NSAIDS, patients undergoing or about to undergo chemotherapy, and patients who suffer from stress-induced ulceration.

Treatment with GLP-2 agonists has been demonstrated to increase tissue growth in the upper gastrointestinal tract. Models suitable for determining which analogs of GLP-2 have upper gastrointestinal tract proliferation activity are potentially therapeutically useful to treat medical or veterinary conditions of the upper gastrointestinal tract are described in example 1 and example 3.

Animal models useful for studying conditions involving the esophagus are described in the literature (see, for example, *Ann. Surg.* Oncol. 1(3):252-261 (1994); *Ann. Emerg. Med.* 22(2):178-182 (1993)) and include mechanical injury or acid-induced injury of the esophagus. Thus, the animal models described in the art can be used to assess the ability of compounds identified as GLP-2 agonists to ameliorate inflammatory conditions involving the upper gastrointestinal tract.

Animal models useful for studying conditions involving the stomach are described in the literature (see, for example, *Experimental and Molecular Pathology* 59:136-154(1993)). These models include administration of non specific anti-inflammatories to cause gastroduodenitis, and administration of ethanol alone or in combination with other agents that cause gastric damage. Thus, the animal models described in the art can be used to assess the ability of compounds identified as GLP-2 agonists to ameliorate conditions involving the stomach.

The various vertebrate forms of GLP-2 include, for example, rat GLP-2 and its homologous including ox GLP-2, porcine GLP-2, degu GLP-2, bovine GLP-2, guinea pig GLP-2, hamster GLP-2, human GLP-2, rainbow trout GLP-2, and chicken GLP-2. The sequences of these forms of GLP-2 have been reported by many authors including Buhl et al. in *J. Biol. Chem.,* 1988, 263(18):8621, Nishi and Steiner, *Mol. Endocrinol*., 1990, 4:1192-8, and Irwin and Wong, *Mol. Endocrinol*., 1995, 9(3):267-77.

Analogs of vertebrate GLP-2 can be generated using standard techniques of peptide chemistry and can be assessed for trophic activity at the upper gastrointestinal tract, all according to the guidance provided herein. Particularly preferred analogs of the invention are those based upon the sequence of human GLP-2, as follows: 
His-Ala-Asp-Gly-Ser-Phe-Ser-Asp-Glu-Met-Asn-Thr-
Ile-Leu-Asp-Asn-Leu-Ala-Ala-Arg-Asp-Phe-Ile-Asn-
Trp-Leu-Ile-Gln-Thr-Lys-Ile-Thr-Asp
 wherein one or more amino acid residues are conservatively substituted for another amino acid residue, as long as the analog still maintains its trophic activity at the upper gastrointestinal tract as measured by an increase in at least one of the following parameters: upper gastrointestinal tract length, protein content, mass or rate of mitosis/cell division of cells of the upper gastrointestinal tract, measured, for example, by the use of an indirect indicator of mitosis such as bromodeoxyuridine(BrDU).

Conservative substitutions in any naturally occurring GLP-2, preferably the human GLP-2 sequence, are defined as exchanges within any of the following five groups:
I. Ala, Ser, Thr, Pro, Gly
II. Asn, Asp, Glu, Gln
III. His, Arg, Lys
IV. Met, Leu, Ile, Val, Cys
V.

Phe, Tyr, Trp.

The invention also encompasses non-conservative substitutions of amino acids in any vertebrate GLP-2 sequence, provided that the non-conservative substitutions occur at amino acid positions known to vary in GLP-2 isolated from different species. Non-conserved residue positions are readily determined by aligning all known vertebrate GLP-2 sequences. For example, Buhl et al., *J. Biol. Chem*., 1988, 263(18):8621, compared the sequences of human, porcine, rat, hamster, guinea pig, and bovine GLP-2's, and found that positions 13, 16, 19, 27 and 28 were non-conserved (position numbers refer to the analogous position in the human GLP-2 sequence). Nishi and Steiner, *Mol. Endocrinol*., 1990, 4:1192-8, found that an additional position within the sequence encoding GLP-2, residue 20 in the above human sequence, also varied in degu, a rodent species indigenous to South America. Thus, under this standard, the amino acid positions which vary in mammals and which preferable may be substituted with non-conservative residues are positions 13, 16, 19, 20, 27 and 28. The additional amino acid residues which vary in vertebrates and which also may be substituted with non-conserved residues occur at positions 2, 5, 7, 8, 9, 10, 12, 17, 21, 22, 23, 24, 26, 29, 30, 31, 32 and 33.

Alternatively, non-conservative substitutions may be made at any position in which alanine-scanning mutagenesis reveals some tolerance for mutation in that substitution of an amino acid residue with alanine does not destroy all activity at the upper gastrointestinal tract. The technique of alanine scanning mutagenesis is described by Cunningham and Wells, *Science,* 1989, 244:1081, and incorporated herein by reference in its entirety. Since most GLP-2 sequences consist of only approximately 33 amino acids (and in human GLP-2 alanine already occurs at four positions), one of skill in the art could easily test an alanine analogue at each remaining position for effect, as taught in the examples below.

In specific embodiments of the invention, the GLP-2 peptide is selected from
1) rat GLP-2 having the sequence illustrated below: 
His-Ala-Asp-Gly-Ser-Phe-Ser-Asp-Glu-Met-Asn-Thr-
Ile-Leu-Asp-Asn-Leu-Ala-Thr-Arg-Asp-Phe-Ile-Asn-
Trp-Leu-Ile-Gln-Thr-Lys-Ile-Thr-Asp;
2) human GLP-2, the Thr¹⁹ to Ala¹⁹ equivalent of rat GLP-2, illustrated below 
His-Ala-Asp-Gly-Ser-Phe-Ser-Asp-Glu-Met-Asn-Thr-
Ile-Leu-Asp-Asn-Leu-Ala-Ala-Arg-Asp-Phe-Ile-Asn-
Trp-Leu-Ile-Gln-Thr-Lys-Ile-Thr-Asp;
3) human [Gly²]GLP-2 (human GLP-2 wherein the alanine at position 2 is replaced by a glycine);
4) GLP-2's, and GLP-2 analogs, which incorporate an N-terminal blocking group and/or an N-terminal extension such as Arg or Arg-Arg; and/or incorporate a C-terminal blocking group and/or a C-terminal extension such as Arg or Arg-Arg.

Further, a large number of agonist GLP-2 peptides that are described in PCT Application PCT/CA97/00252, corresponding to EP97916280.7, filed April 11, 1997 may also be used in the methods of the invention.

The "blocking groups" represented by R1 and R2 are chemical groups that are routinely used in the art of peptide chemistry to confer biochemical stability and resistance to digestion by exopeptidase. Suitable N-terminal protecting groups include, for example, C₁₋₅ alkanoyl groups such as acetyl. Also suitable as N-terminal protecting groups are amino acid analogues lacking the amino function. Suitable C-terminal protecting groups include groups which form ketones or amides at the carbon atom of the C-terminal carboxyl, or groups which form esters at the oxygen atom of the carboxyl. Ketone and ester-forming groups include alkyl groups, particularly branched or unbranched C₁₋₅ alkyl groups, e.g., methyl, ethyl, and propyl groups, while amide-forming groups include amino functions such as primary amine, or alkylamine functions, e.g., mono-C₁₋₅alkylamino and di-C₁₋₅alkylamino groups such as methylamino, ethylamino, dimethylamino, diethylamino, methylethylamino and the like. Amino acid analogues are also suitable for protecting the C-terminal end of the present compounds, for example, decarboxylated amino acid analogues such as agmatine.

GLP-2 peptides useful herein include derivatized forms of the GLP-2 species just described, in which various functional groups are coupled to the peptide, for instance, to prolong half life, enhance solubility, direct GLP-2 action, etc. In one embodiment, GLP-2 is modified at existing or substituted amino acids, such as lysine and arginine, to incorporate a lipophilic moiety for purposes of prolonging half-life. As described in WO98/08872, published March 5, 1998, such lipophilic moieties include the tetradecanoyl group and the carboxynonadecanoyl group, attached, for example, at the epsilon amino group of an additional C-terminal lysine or at a lysine replacement amino acid at position 20 of GLP-2(1-33).

The particular form of GLP-2 selected for promoting the growth of upper gastrointestinal tract tissue can be prepared by a variety of techniques well known for generating peptide products. Vertebrate forms of GLP-2 can of course be obtained by extraction from the natural source, using an appropriate combination of protein isolation techniques. As described by Buhl et al., *supra,* porcine GLP-2 isolation and purification is achieved from acid-ethanol extracts of ileal mucosa by a combination of size selection and HPLC-based fractionation, with the aid of antibody raised against synthetic proglucagon 126-159, to monitor work-up. As an alternative to GLP-2 extraction, those forms of GLP-2 that incorporate only L-amino acids, whether vertebrate GLP-2 or analogs thereof, can be produced in commercial quantities by application of recombinant DNA technology. For this purpose, DNA coding for the desired GLP-2 or GLP-2 analog is incorporated into an expression vector and transformed into a microbial, *e.g.*, yeast, or other cellular host, which is then cultured under conditions appropriate for GLP-2 expression. A variety of gene expression systems have been adapted for this purpose, and typically drive expression of the desired gene from expression controls used naturally by the chosen host. Because GLP-2 does not require post translational glycosylation for its activity, its production may most conveniently be achieved in bacterial hosts such as *E. coli*. For such production, DNA coding for the selected GLP-2 peptide may usefully be placed under expression controls of the lac, trp or PL genes of *E. coli*. As an alternative to expression of DNA coding for the GLP-2 per se, the host can be adapted to express GLP-2 peptide as a fusion protein in which the GLP-2 is linked releasable to a carrier protein that facilitates isolation and stability of the expression product.

In an approach universally applicable to the production of a selected GLP-2 or GLP-2 analog, and one used necessarily to produce GLP-2 peptides that incorporate non-genetically encoded amino acids and N- and C-terminally derivatized forms, the well established techniques of automated peptide synthesis are employed, general descriptions of which appear, for example, in J. M. Stewart and J. D. Young, Solid Phase Peptide Synthesis, 2nd Edition, 1984 Pierce Chemical Company, Rockford, Illinois; and in M. Bodanszky and A. Bodanszky, The Practice of Peptide Synthesis, 1984, Springer-Verlag, New York; Applied Biosystems 430A Users Manual, 1987, ABI Inc. Foster City, California. In these techniques, GLP-2 peptide is grown from its C-terminal, resin-conjugated residue by the sequential addition of appropriately protected amino acids, using either the Fmoc or tBoc protocols, as described for instance by Orskov et al., *Febs Letters,* 1989, 247(2):193-196.

For the incorporation of N- and/or C- blocking groups, protocols conventional to solid phase peptide synthesis methods can also be applied. For incorporation of C-terminal blocking groups, for example, synthesis of the desired peptide is typically performed using, as solid phase, a supporting resin that has been chemically modified so that cleavage from the resin results in a GLP-2 peptide having the desired C-terminal blocking group. To provide peptides in which the C-terminus bears a primary amino blocking group, for instance, synthesis is performed using a p-methylbenzhydrylamine (MBHA) resin so that, when peptide synthesis is completed, treatment with hydrofluoric acid releases the desired C-terminally aminated peptide. Similarly, incorporation of an N-methylamine blocking group at the C-terminus is achieved using N-methylaminoethyl-derivatized DVB resin, which upon HF treatment releases peptide bearing an N-methylamidated C-terminus. Protection of the C-terminus by esterification can also be achieved using conventional procedures. This entails use of resin/blocking group combination that permits release of side-chain protected peptide from the resin, to allow for subsequent reaction with the desired alcohol, to form the ester function. FMOC protecting groups, in combination with DVB resin derivatized with methoxyalkoxybenxyl alcohol or equivalent linker, can be used for this purpose, with cleavage from the support being effected by TFA in dichloromethane. Esterification of the suitably activated carboxyl function, *e.g*., with DCC, can then proceed by addition of the desired alcohol, followed by deprotection and isolation of the esterified GLP-2 peptide.

Incorporation of N-terminal blocking groups can be achieved while the synthesized GLP-2 peptide is still attached to the resin, for instance by treatment with suitable anhydride and nitrile. To incorporate an acetyl blocking group at the N-terminus, for instance, the resin-coupled peptide can be treated with 20% acetic anhydride in acetonitrile. The N-blocked GLP-2 peptide can then be cleaved from the resin, deprotected and subsequently isolated.

Once the desired GLP-2 peptide has been synthesized, cleaved from the resin and fully deprotected, the peptide is then purified to ensure the recovery of a single oligopeptide having the selected amino acid sequence. Purification can be achieved using any of the standard approaches, which include reversed-phase high-pressure liquid chromatography (RP-HPLC) on alkylated silica columns, *e.g*., C₄-, C₈-, or C₁₈- silica. Such column fractionation is generally accomplished by running linear gradients, *e.g*., 10 - 90%, of increasing % organic solvent, *e.g*., acetonitrile, in aqueous buffer, usually containing a small amount *(e.g.,* 0.1%) of pairing agent such as TFA or TEA. Alternatively, ion-exchange HPLC can be employed to separate peptide species on the basis of their charge characteristics. Column fractions are collected, and those containing peptide of the desired/required purity are optionally pooled. In one embodiment of the invention, the GLP-2 peptide is then treated in the established manner to exchange the cleavage acid (*e.g*., TFA) with a pharmaceutically acceptable acid, such as acetic, hydrochloric, phosphoric, maleic, tartaric, succinic and the like, to generate a pharmaceutically acceptable acid addition salt of the peptide.

For administration to patients, the GLP-2 peptide or its salt is provided, in one aspect of the invention, in pharmaceutically acceptable form, *e.g.,* as a preparation that is sterile-filtered, *e.g*., through a 0.22µ filter, and substantially pyrogen-free. Desirably, the GLP-2 peptide to be formulated migrates as a single or individualized peak on HPLC, exhibits uniform and authentic amino acid composition and sequence upon analysis thereof, and otherwise meets standards set by the various national bodies which regulate quality of pharmaceutical products.

For therapeutic use, the chosen GLP-2 or GLP-2 analog is formulated with a carrier that is pharmaceutically acceptable and is appropriate for administering the peptide to the subject by the chosen route of administration so as to deliver the peptide to the upper gastrointestinal tract. Suitable pharmaceutically acceptable carriers are those used conventionally with peptide-based drugs, such as diluents, excipients and the like. Reference may be made to "Remington's Pharmaceutical Sciences", 17th Ed., Mack Publishing Company, Easton, Penn., 1985, for guidance on drug formulations generally. In one embodiment of the invention, the compounds are formulated for administration by infusion, *e.g*., when used as liquid nutritional supplements for patients on total parenteral nutrition therapy, or by injection, *e.g*., sub-cutaneously, intramuscularly or intravenously, and are accordingly utilized as aqueous solutions in sterile and pyrogen-free form and optionally buffered to physiologically tolerable pH, *e.g*., a slightly acidic or physiological pH. Thus, the compounds may be administered in a vehicle such as distilled water or, more desirably, in saline, phosphate buffered saline or 5% dextrose solution. Water solubility of the GLP-2 or GLP-2 analog may be enhanced, if desired, by incorporating a solubility enhancer, such as acetic acid or sodium hydroxide.

The aqueous carrier or vehicle can be supplemented for use as injectables with an amount of gelatin effective to achieve the depot effect are expected to lie in the range from 10-20%. Alternative gelling agents, such as hyaluronic acid, may also be useful as depoting agents (also veterinary applications).

As an alternative to injectable formulations, the GLP-2 or GLP-2 analog may be formulated for administration to patients and delivery to the upper gastrointestinal tract by other routes. Oral dosage forms, such as tablets, capsules and the like, can be formulated in accordance with standard pharmaceutical practice.

The compounds may also be formulated in liquid form to provide direct contact with the esophagus and stomach, in lozenge form or other similar formulations which permit direct contact with the lining of the esophagus or stomach and the compound. Further, standard formulations may be delivered to achieve rapid and high levels of the compound in the circulation.

The GLP-2's and GLP-2 analogs of the invention may also be formulated as a slow release implantation device for extended and sustained administration of GLP-2. Examples of such sustained release formulations include composites of bio-compatible polymers, such as poly(lactic acid), poly(lactic-co-glycolic acid), methylcellulose, hyaluronic acid, collagen, and the like. The structure, selection and use of degradable polymers in drug delivery vehicles have been reviewed in several publications, including, A. Domb et al., *Polymers for Advanced Technologies* 3:279-292 (1992). Additional guidance in selecting and using polymers in pharmaceutical formulations can be found in the text by M. Chasin and R. Langer (eds.), "Biodegradable Polymers as Drug Delivery Systems", Vol. 45 of "Drugs and the Pharmaceutical Sciences", M. Dekker, New York, 1990. Liposomes may also be used to provide for the sustained release of a GLP-2 or GLP-2 analog. Details concerning how to use and make liposomal formulations of drugs of interest can be found in, among other places, U.S. Pat. No. 4,921,706; U.S. Pat. No. 5,008,050; U.S. Pat. No. 4,921,706; U.S. Pat. No. 4,927,637; U.S. Pat. No. 4,452,747; U.S. Pat. No. 4,016,100; U.S. Pat. No. 4,311,712; U.S. Pat. No. 4,370,349; U.S. Pat. No. 4,372,949; U.S. Pat. No. 4,529,561; U.S. Pat. No. 5,009,956; U.S. Pat No. 4,725,442; U.S. Pat. No. 4,737,323; U.S. Pat. No. 4,920,016. Sustained release formulations are of particular interest when it is desirable to provide a high local concentration of a GLP-2 or GLP-2 analog, *e.g*., near or at the upper gastrointestinal tract to promote upper gastrointestinal tract growth in the inflamed upper gastrointestinal tract.

For use in stimulating growth of the upper gastrointestinal tract, and/or enhancing upper gastrointestinal tract functioning in a mammal including a human, the present invention provides in one of its aspects a package, in the form of a sterile-filled vial or ampoule, that contains a tissue growth promoting amount of the GLP-2 or GLP-2 analog, in either unit dose or multi-dose amounts, wherein the package incorporates a label instructing use of its contents for the promotion of such growth. In one embodiment of the invention, the package contains the GLP-2 or GLP-2 analog and the desired carrier, as an administration-ready formulation. Alternatively, and according to another embodiment of the invention, the package provides the GLP-2 or GLP-2 analog in a form, such as a lyophilized form, suitable for reconstitution in a suitable carrier, such as phosphate-buffered saline.

In one embodiment, the package is a sterile-filled vial or ampoule containing an injectable solution which comprises an effective, upper gastrointestinal tract proliferating amount of GLP-2 or GLP-2 analog dissolved in an aqueous vehicle.

According to the present invention, the GLP-2 or GLP-2 analog is administered to treat patients that would benefit from growth of the tissue of the upper gastrointestinal tract. In addition, patients who would benefit from increased upper gastrointestinal tract tissue function, whether as a result of increased tissue growth or not, are candidates for treatment with the invention. In general, patients who would benefit from either increased upper gastrointestinal tract mass and/or increased upper gastrointestinal tract mucosal function are candidates for treatment with GLP-2 or GLP-2 analog. Particular conditions that may be treated with GLP-2 include the various forms of inflammatory diseases of the stomach or esophagus, as well as patients who have undergone partial or sub-total resection of the upper gastrointestinal tract. For convenience a non-exhaustive list of conditions of the upper gastrointestinal tract including the stomach and esophagus, that may be treated by the subject GLP-2 or in combinations, is provided in the following tables.

**TABLE 1**

| Disorders of the Stomach | | | | |
|---|---|---|---|---|
| I. | Acute Gastritis | | | |
| | | | | |
| | A. | Acute hemorrhagic and erosive gastritis | | |
| | | 1. | Acute erosive gastritis | |
| | | 2. | Acute hemorrhagic gastritis | |
| | | 3. | Acute gastritis | |
| | | 4. | Acute stress gastritis | |
| | | 5. | Nonsteroidal anti-inflammatory drug | |
| | | 6. | Gastropathy (acute) | |
| | | 7. | Hemorrhagic gastropathy | |
| | | | | |
| | B. | Acute *Helicobacter pylori* gastritis | | |
| | | 1. | Type B gastritis | |
| | | 2. | Hypersecretory gastritis | |
| | | 3. | Non specific gastritis secondary to *Helicobacter pylori* | |
| | | 4. | *Helicobacter pylori* - associated gastritis | |
| | | | | |
| | C. | Chemical gastritis | | |
| | | 1. | Type C gastritis (i.e., chemical) | |
| | | 2. | Reactive gastritis | |
| | | 3. | Reflux gastritis | |
| | | 4. | Bile gastritis | |
| | | 5. | Nonsteroidal anti-inflammatory drug gastropathy (chronic) | |
| | | | | |
| | D. | Other acute infectious gastritis (See Uncommon Forms of Gastritits) | | |
| | | | | |
| II. | Common Forms of Chronic Gastritis | | | |
| | | | | |
| | A. | *Helicobactor pylori* gastritis | | |
| | B. | Chemical gastritis | | |
| | | 1. | Bile reflux | |
| | | 2. | Type C gastritis (i.e., chemical) | |
| | | 3. | Reactive gastritis | |
| | | 4. | Reflux gastritis | |
| | | 5. | Aspirin and nonsteroidal anti-inflammatory drug gastropathy (chronic) | |
| | | | | |
| | C. | Metaplastic atrophic gastritis | | |
| | | 1. | Autoimmune Metaplastic atrophic gastritis | |
| | | | a. | Atrophic gastritis |
| | | | b. | Type A gastritis |
| | | | c. | Diffuse corporal gastritis |
| | | | d. | Autoimmune chronic gastritis |
| | | | e. | Autoimmune-associated gastritis |
| | | | | |
| | | 2. | Environmental metaplastic atrophic gastritis | |
| | | | a. | Environmental chronic gastritis |
| | | | b. | Multifocal environmental gastritis |
| | | | c. | Multifocal atrophic gastritis |
| | | | d. | Atrophic gastritis |
| | | | e. | Chronic atrophic gastritis |
| | | | f. | Type B gastritis |
| | | | g. | Idiopathic pangastritis |
| | | | | |
| III. | Uncommon Forms of gastritis | | | |
| | | | | |
| | A. | Postantrectomy atrophic gastritis | | |
| | B. | Eosinophilic gastritis | | |
| | C. | Infectious gastritis | | |
| | | 1. | Bacterial (other than *Helicobacter pylori*) | |
| | | | a. | *Gastrospirillum hominis* |
| | | | b. | Phlegmonous |
| | | | c. | Mycobacterial |
| | | | d. | Syphiltic |
| | | | | |
| | | 2. | Viral | |
| | | 3. | Parasitic | |
| | | 4. | Fungal | |
| | | | | |
| | D. | Crohn's disease | | |
| | E. | Sarcoidosis | | |
| | F. | Isolated granulomatous gastritis | | |
| | G. | Lymphocylic gastritis | | |
| | H. | Ménétriere's disease | | |
| See, *Gastritis, Duodenitis, and Associated Ulcerative Lesions*, by John H. Yardley and Thomas R. Hendrix, in *Textbook on Gastroenterology* Vol. I, edited by Tadataka Yamada M.D. (2nd ed. 1985). | | | | |

**TABLE 2**

| Disorders of the Esophagus | | | | |
|---|---|---|---|---|
| I. | Infectious Esophagitis | | | |
| | | | | |
| | A. | Organisms associated with infections | | |
| | | 1. | Fungi | |
| | | | a. | *Candida* species (esp. *albicans)* |
| | | | b. | *Aspargillus* species |
| | | | c. | *Histoplasma capsulatum* |
| | | | d. | *Blastomyces dermatitides* |
| | | | | |
| | | 2. | Viruses | |
| | | | a. | Herpes simplex virus (type 1) |
| | | | b. | Cytomegalovirus |
| | | | c. | Varicella-zoster virus |
| | | | | |
| | | 3. | Bacteria | |
| | | | a. | *Mycobacterium tuberculosis* |
| | | | b. | *Actinomyces Israelii* |
| | | | c. | *Streptococcus viridans* |
| | | | d. | *Lactobacillus acidophilus* |
| | | | e. | *Treponema pallidum* |
| | | | | |
| II. | Non-infectious Esophagitis | | | |
| | | | | |
| III. | Acid Reflux | | | |
| | | | | |
| IV. | Bile Reflux | | | |
| | | | | |
| V. | Chemical Injury | | | |
| | | | | |
| | A. | Medicines | | |
| | B. | Toxins | | |
| | C. | Acids | | |
| | D. | Alkali | | |
| | | | | |
| VI. | Sarcoidosis | | | |
| | | | | |
| VII. | Crohn's disease | | | |
| | | | | |
| VIII. | | Behcet's disease | | |
| | | | | |
| IX. | Graft-versus-host disease | | | |
| | | | | |
| X. | AIDS Related Infections | | | |
| | | | | |
| | A. | *Cryptosporidium sp* | | |
| | | *Microsporidium sp* | | |
| | | *Isospora beill* | | |
| | | *Glardia Lamblia* | | |
| | | *Salmonella sp* | | |
| | | *Shigella sp* | | |
| | | *Campylobacter sp* | | |
| | | *Mycobacterium tuberculosis* | | |
| | | *Mycobacterium avium complex (MAC)* | | |
| | | *Clostridium difficile* | | |
| | | *Cytomeglavorius* | | |
| | | *Herpes simplex* | | |
| See, *Miscellaneous Diseases of the Esophagus,* in *Textbook on Gastroenterology* Vol. I, edited by Tadataka Yamada M.D. (2nd ed. 1985). | | | | |

The therapeutic efficacy of the GLP-2 treatment on the esophagus may be monitored by, for example, endoscopy, barium swallow or CT. For example, GLP-2 or GLP-2 analog is administered to a patient with an inflammatory condition involving the upper gastrointestinal tract in an amount sufficient to ameliorate the esophageal discomfort, decrease pain, improve swallowing, reduce chest pain, decrease heartburn, decrease regurgitation of solids or liquids after swallowing or eating, decrease in vomiting, or improve weight gain or improve vitality. The therapeutic efficacy of GLP-2 on the stomach amy be monitored by a decrease in pain or discomfort. Additionally, GLP-2 or GLP-2 analog may be administered to patients who are identified as being at risk of developing an inflammatory condition involving the upper gastrointestinal tract such as patients treated with nonsteroidal anti-inflammatories ("NSAIDS"). A few examples of NSAIDS are aspirin, ibuprofen, sulindac, and indomethocin.

The therapeutic dosing and regimen most appropriate for patient treatment will of course vary with the disease or condition to be treated, and according to the patient's weight and other parameters. The results presented hereinbelow in Examples 1-3 demonstrate that treatment with GLP-2 or GLP-2 analog administered twice daily over 2 days results in cell proliferation and an increase in protein synthesis of the upper gastrointestinal tract. Treatment for 12 days resulted in an increase in stomach mass. Example 4 demonstrates that GLP-2 treatment is able to ameliorate the gastrointestinal toxicity of nonsteroidal anti-inflammatory drugs. It is expected that much smaller doses, and shorter or longer duration or frequency of treatment, will also produce therapeutically useful results, *i.e*., a statistically significant increase particularly in upper gastrointestinal tract mass, and/or enhanced upper gastrointestinal tract functioning. The dosage sizes and dosing regimen most appropriate for human use are guided by the results herein presented, and can be confirmed in properly designed clinical trials.

An effective dosage and treatment protocol may be determined by conventional means, starting with a low dose in laboratory animals and then increasing the dosage while monitoring the effects, (*e.g*., histology, disease activity scores, mucosal enzyme activity, small and large bowel epithelial-specific gene expression, markers of the inflammatory response such as expression of cytokines and myeloperoxidase, GLP-2 content) and systematically varying the dosage regimen as well. Numerous factors may be taken into consideration by a clinician when determining an optimal dosage for a given subject. Primary among these is the amount of GLP-2 normally circulating in the plasma, which is on the order of 151 pmol/mL in the resting state, rising to 225 pmol/mL after nutrient ingestion for healthy adult humans (Orskov, C. and Holst, J. J., 1987, *Scand. J. Clin. Lab. Invest.* 47:165). Additional factors include the size of the patient, the age of the patient, the general condition of the patient, the particular disease being treated, the severity of the disease, the presence of other drugs in the patient, the *in vivo* activity of the GLP-2 peptide and the like. The trial dosages would be chosen after consideration of the results of animal studies and the clinical literature. It will be appreciated by the person of ordinary skill in the art that information such as binding constants and Ki derived from *in vitro* GLP-2 binding competition assays may also be used in calculating dosages.

A typical human dose of a GLP-2 peptide would be from about 10 µg/kg body weight/day to about 10 mg/kg/day, preferably from about 50 µg/kg/day to about 5 mg/kg/day, and most preferably about 100 µg/kg/day to 1 mg/kg/day.

In another of its aspects, the invention provides for the treatment of patient candidates as just identified using implanted cells that have either been conditioned *in vitro* or *in vivo* by prior incubation or treatment with GLP-2 or GLP-2 analog, or have been engineered genetically to produce it. Conditioning of the cells *ex vivo* can be achieved simply by growing the cells or tissue to be transplanted in a medium that has been supplemented with a growth-promoting amount of the GLP-2 or GLP-2 analog and is otherwise appropriate for culturing of those cells. The cells can, after an appropriate conditioning period, then be implanted either directly into the patient or can be encapsulated using established cell encapsulation technology, and then implanted.

Yet another aspect of the invention encompasses treating animals *in vivo* with GLP-2 peptides in order to promote the growth of esophageal tissue. After subsequent enlargement of the upper gastrointestinal tract these tissues may then be used in a xenotransplantation procedure. Such GLP-2 peptide treatment can be advantageous prior to xenotransplantation of the tissue from a non-human animal to a human because the size of the transplanted organ or tissue often limits the success of this procedure. For example, a porcine donor animal may be treated with GLP-2 peptide in order to increase upper gastrointestinal tract size prior to xenotransplantation of the porcine upper gastrointestinal tract tissue into a human in need of this organ.

Alternatively, the cells to be implanted can be raised *in vitro* from a cell that has been engineered genetically to express or to over-express either the glucagon gene or, more directly, DNA coding solely for GLP-2. The sequence of such DNA can readily be determined from the amino acid sequence of the selected GLP-2, with the limitation that only GLP-2 forms containing genetically encoded amino acids can be produced in this manner. Various viral vectors, suitable for introduction of genetic information into human cells, can be employed and will incorporate the GLP-2-encoding DNA under expression controls functional in the host cells. Once altered genetically, the engineered cells can then be implanted using procedures established in the art. (*See*, for example, Drucker et al., 1996, *PNAS:USA* 93:7911-7916.)

### Coadministration of GLP-2 and Other Peptide Hormones

The invention relates to therapeutically useful combinations of GLP-2 and at least one other peptide hormone selected from the group consisting of IGF-1, IGF-2, and GH. Surprisingly, GLP-2 (and its analogs) administered alone to a mammal induces the growth of the upper gastrointestinal tract. As predicted, when GLP-2 is administered in combination with at least one other peptide hormone selected from the group IGF-1, IGF-2, and GH, there are clear effects on the growth of upper gastrointestinal tract tissue are seen.

The GLP-2's and GLP-2 analogs of the invention may also be administered to a subject in admixture with at least one other peptide hormone which is known to be an esophageal or gastric growth factor. Known esophageal and gastric growth factors include epidermal growth factor (EGF), insulin-like growth factor 1 (IGF-1), hepatocyte growth factor (HGF) and keratinocyte growth factor (KGF).

Specific adjunct therapies with GLP-2 useful for the treatment of conditions involving the stomach, *e.g*., peptidic ulcer include: (1) agents that act to block acid secretion from the stomach, *e.g*., H2 receptor antagonists, proton pump inhibitors and prostaglandins; (2) agents that act to form a protective barrier in the stomach, *e.g*., sucralfate; (3) bismuth containing compounds, and (4) agents that control *Helicobacter pylori.*

More particularly, the invention relates to the therapeutic and related uses of GLP-2 when co-administered with at least one other peptide hormone selected from the group consisting of IGF-1 and GH. Even more particularly, the invention relates to the uses of the above-mentioned combinations to enhance upper gastrointestinal tract functioning. Most particularly, the invention relates to the therapeutic and related uses of the above-mentioned combinations to promote the proliferation of the mucosal epithelial and muscle cells of the upper gastrointestinal tract.

Unless otherwise specified, the term "GLP-2" refers collectively herein to the various naturally produced forms of GLP-2, particularly mammalian forms. The invention also encompasses those analogs of GLP-2 which exhibit activity. Analogs of GLP-2 can be tested for activity using the mouse model described herein and in co-pending application U.S. Serial No. 08/631,273. Briefly, this testing involves a 10 to 14 day regimen of twice a day (b.i.d) subcutaneous injection of 2.5 mg of the GLP-2 analog (in PBS) per kg body weight with control matched untreated animals receiving PBS alone. Alternatively, the analogs may be administered once a day, or every other day. Upon completion of the regimen the animals are sacrificed and their upper gastrointestinal tracts are removed and weighed. In this manner, the effect of analogs of GLP-2 on the upper gastrointestinal tract can be assessed.

Guidance on particular analogs and variants of GLP-2 that may be usefully employed in the present invention, and guidance on how to produce others, is provided in co-pending applications U.S. Serial Nos. 08/632,533 and 08/631,273, both filed on April 12, 1996, the disclosures of which are incorporated herein by reference. Briefly, any substitution, addition or deletion of GLP-2 that does not destroy the activity of GLP-2 may be usefully employed in this invention. In preferred embodiments the GLP-2 analogs are at least as as native human GLP-2. In the most preferred embodiments, the GLP-2 analog has enhanced activity compared with native human GLP-2. For example, such analogs may exhibit enhanced serum stability, enhanced receptor binding and enhanced signal transducing activity. Other modifications to GLP-2 and GLP-2 analogs that may usefully be employed in this invention are those which render the molecule resistant to oxidation.

The GLP-2 analogs are suitably analogs of either human GLP-2 (human [Gly²] GLP-2) or rat GLP-2 (rGLP-2). In a preferred embodiment of the invention, rat or human GLP-2 is altered at position 2 to confer DPP-IV resistance by substituting a Gly for an Ala. Human GLP-2 having Gly substituted for Ala at position 2 is referenced herein as [Gly 2] human [Gly²] GLP-2.

Similarly, the terms "IGF-1", "IGF-2" and "GH" as used herein encompass effective analogs and variants of the naturally produced peptides as well as the native peptides. Guidance on particular analogs and variants of IGF-1, IGF-2, and GH that may usefully be employed in the present invention is provided in the following publications which are incorporated herein by reference: Vanderhoof et al. (1992) *Gastroenterology* 102:1949-1956; Steeb et al. (1994) *Am. J. Physiol.* 266:G1090-G1098; and Jones et al. (1995) *Endocrine Reviews* 16:3-34; Conlon et al. (1995) *Journal of Endocrinology* 146:247-253; Francis et al. (1993) *Biochemical Journal* 293:713-719; Lewis et al., WO 93/20836; and Bozyczko-Coyne et al., WO 93/08826.

Secretagogues, factors capable of enhancing endogenous production, of the subject peptide hormones may also be included as part of a therapeutic regimen, either in lieu of or as a supplement to the subject peptide hormone the release of which it stimulates. Thus, the inclusion of such factors, which are well known to those of ordinary skill in the art, is within the scope of the invention. For example, production of endogenous GH is increased by growth hormone releasing factor (GHRF) and by arginine. Similarly, the skilled artisan will be aware that compounds such as small molecules, which act on the appropriate receptor to cause an increase in the serum levels of any of the subject peptide hormone, could be usefully employed in the present invention.

As used herein, a compound, such as a peptide, is said to be "co-administered" or in "combination" with another compound when either the physiological effects of both compounds, or the elevated serum concentration of both compounds can be measured simultaneously. With compounds that increase the level of endogenous production, the serum concentration of the endogenously produced hormone and the other administered agent can also be measured simultaneously when "co-administered" or in "combination". Thus, compounds may be administered either simultaneously, as separate or mixed compositions, or they may be administered sequentially provided that a constant elevation of their levels in serum results.

Unless otherwise stated the terms "combination therapy" and "combination treatments" are used herein to describe a therapeutic regimen involving co-administration of the subject peptide hormones which results in an enhancement of the nutritional status, or an increase in intestinal mass, of a patient.

As used herein, the terms "enhanced nutritional status" and "enhanced gut functioning" are defined as any increase in bodily uptake of nutrients over pretreatment levels. Such nutrients include, but are not limited to, carbohydrates, protein and amino acids, fat, cholesterol and fat-soluble vitamins, water soluble vitamins, and minerals. Minerals, the uptake of which can be increased by the methods of the present invention, include, but are not limited to, Na, Ca, Mg, K, Zn, and Fe. Vitamins, the absorption of which can be increased by the present invention include fat soluble vitamins such as vitamins A, D, E and K as well as water soluble vitamins such as B¹² and folic acid.

As used herein the term "patient" is intended to include, but is not limited to humans, livestock and pets.

A mammal is said to be suffering from disorders, diseases and medical conditions of the gut when the absorptive properties of the gut of the mammal are diminished, and/or when inflammation or injury to the gastrointestinal tract is present so as to cause discomfort and illness. A variety of tests can be employed to determine if a mammal is suffering from malabsorption syndrome. These include stool fat content, xylose absorption, gastrointestinal x-ray studies, small intestine biopsy test, the Schilling test for vitamin B¹² absorption and the secretin test.

In one aspect of the invention, GLP-2 is provided for administration to patients in admixture with at least one other peptide hormone selected from the group consisting of IGF-1, IGF-2, and GH in pharmaceutically acceptable form (for example, as a preparation that is sterile filtered through a 0.22 µm filter and substantially pyrogen free). Desirably, the peptides to be admixed migrate as single peaks on HPLC.

The particular forms of GLP-2, IGF-1, IGF-2 and GH selected for the invention can be prepared by a variety of techniques well known for generating peptide products. Those forms of GLP-2, IGF-1, IGF-2 and GH that occur naturally can of course be obtained by extraction from the natural source, using an appropriate combination of protein isolation techniques. For example, as described by Buhl et al., (1988) *J. Bio. Chem.* 263(18):8621-8624, porcine GLP-2 isolation and purification is achieved from acid-ethanol extracts of ileal mucosa by a combination of size selection and HPLC-based fractionation, with the aid of antibody raised against synthetic proglucagon 126-159, to monitor work-up. Similarly, GH can be extracted from cadavers as described in U.S. Patent No. 2,974,088.

As an alternative to extraction, those forms of GLP-2, IGF-1, IGF-2 and GH that incorporate only L-amino acids can be produced reproducibly and in commercial quantities by application of recombinant DNA technology. For this purpose, nucleic acids coding for the desired form of GLP-2, IGF-1 (see, for example, U.S. Patent No. 5,288,931), IGF-2 and GH (see, for example, Goeddel et al. (1979) *Nature* 281:544-548) are incorporated expressibly in a cellular host, which is then cultured under conditions appropriate for expression of that particular peptide or protein. A variety of gene expression systems have been adapted for this purpose, and typically drive expression of the desired gene from expression controls used naturally by the chosen host. Because GLP-2, IGF-1, IGF-2 and GH do not require post translational modification for activity, their production may conveniently be achieved in bacterial hosts such as *E. coli.* For such production, DNA coding for the selected GLP-2, IGF-1, IGF-2 or GH may usefully be placed under expression controls of the lac, trp or PL genes of *E. coli.* As an alternative to expression of DNA coding for the GLP-2, IGF-1, IGF-2 or GH per se, the host can be adapted to express GLP-2, IGF-1, IGF-2 or GH as a fusion protein in which the GLP-2, IGF-1, IGF-2 or GH is linked releasably to a carrier protein that facilitates isolation and stability of the expression product.

For therapeutic use, the peptide hormones chosen for use in combination therapy are formulated with at least one carrier that is pharmaceutically acceptable and is appropriate for delivering the peptides by the chosen route of administration. Suitable pharmaceutically acceptable carriers are those used conventionally with peptide-based drugs, such as diluents, excipients and the like. Reference may be made to "Remington's Pharmaceutical Sciences", 17th Ed., Mack Publishing Company, Easton, Penn., 1985, for guidance on drug formulations generally. In one embodiment of the invention, the compounds are formulated for administration by infusion or by injection, either sub-cutaneously or intravenously, and are accordingly utilized as aqueous solutions in sterile and pyrogen-free form and optionally buffered to a slightly acidic or physiological pH. Thus, the compounds may be administered in distilled water or, more desirably, in saline, buffered saline or 5% dextrose solution. Water solubility of these compounds may be enhanced, if desired, by incorporating a solubility enhancer, such as the inclusion of acetic acid for GLP-2 formulations.

The subject invention also provides for various peptide hormone conjugates. The peptide hormone compositions of the invention comprise peptide hormone covalently linked to one or more water soluble polymers. Water soluble polymers, especially polyethylene glycol, have been conjugated to proteins so as to provide additional desirable properties while retaining, at least in part, the growth inducing properties of the peptide hormone. These desirable properties include increased solubility in aqueous solutions, increased stability in storage, reduced immunogenicity, increased resistance to proteolytic degradation, and increased *in vivo* half-life. Water soluble polymers suitable for use in the subject compositions include polyethylene glycol homopolymers, polypropylene glycol homopolymers, copolymers of ethylene glycol with propylene glycol, wherein said homopolymers and copolymers are unsubstituted or substituted at one end with an alkyl group, polyoxethylated polyols, polyvinyl alcohol, polysaccharides, polyvinyl ethyl ethers, and α,β-poly[(2-hydroxyethyl)-DL-aspartamide]. Polyethylene glycol is particularly preferred. Methods of making water-soluble polymer conjugates of proteins are described in, among other places, U.S. Pat. No. 4,179,337; U.S. Pat. No. 4,609,546; U.S. Pat. No. 4,261,973; U.S. Pat. No. 4,055,635; U.S. Pat. No. 3,960,830; U.S. Pat. No. 4,415,665; U.S. Pat. No. 4,412,989; U.S. Pat. No. 4,002,531; U.S. Pat. No. 4,414,147; U.S. Pat. No. 3,788,948; U.S. Pat. No. 4,732,863; U.S. Pat. No. 4,745,180; EP No. 152,847; EP No. 98,110 (published January 11, 1984); JP No. 5,792,435.

Another aspect of the invention is formulations that provide for the sustained release of peptide hormones. Examples of such sustained release formulations include composites of biocompatible polymers, such as poly(lactic acid), poly(lactic-co-glycolic acid), methylcellulose, hyaluronic acid, collagen, and the like. The structure, selection and use of degradable polymers in drug delivery vehicles have been reviewed in several publications, including, A. Domb et al. (1992) *Polymers for Advanced* *Technologies* 3:279-292. Additional guidance in selecting and using polymers in pharmaceutical formulations can be found in the text by M. Chasin and R. Langer (eds.), "Biodegradable Polymers as Drug Delivery Systems," Vol. 45 of "Drugs and the Pharmaceutical Sciences," M. Dekker, New York, 1990. Liposomes may also be used to provide for the sustained release of peptide hormones. Details concerning how to use and make liposomal formulations of drugs of interest can be found in, among other places, U.S. Pat. No 4,944,948; U.S. Pat. No. 5,008,050; U.S. Pat. No. 4,921,706; U.S. Pat. No. 4,927,637; U.S. Pat. No. 4,452,747; U.S. Pat. No. 4,016,100; U.S. Pat. No. 4,311,712; U.S. Pat. No. 4,370,349; U.S. Pat. No. 4,372,949; U.S. Pat. No. 4,529,561; U.S. Pat. No. 5,009,956; U.S. Pat. No. 4,725,442; U.S. Pat. No. 4,737,323; U.S. Pat. No. 4,920,016. Sustained release formulations are of particular interest when it is desirable to provide a high local concentration of the compositions of the invention, *e.g*., near or in the upper gastrointestinal tract.

For use in stimulating upper gastrointestinal growth and/or improving upper gastrointestinal tissue function in a mammal including a human, the present invention provides in one of its aspects a package, in the form of a sterile-filled vial or ampoule, that contains a tissue growth promoting amount of GLP-2 in admixture with at least one other peptide hormone selected from the group consisting of IGF-1, IGF-2 and GH in either unit dose or multi-dose amounts, wherein the package incorporates a label instructing use of its contents for the promotion of gastrointestinal functioning, *e.g*., for promotion of growth of the upper gastrointestinal tract. In one embodiment of the invention, the package contains GLP-2 and at least one other peptide hormone selected from the group consisting of IGF-1, IGF-2 and GH, and the desired carrier, as an administration-ready formulation. Alternatively, and according to another embodiment of the invention, the package provides the GLP-2 and at least one other peptide hormone selected from the group consisting of IGF-1, IGF-2 and GH in a form, such as a lyophilized form, suitable for reconstitution in a suitable carrier, such as buffered saline. In yet another embodiment, the package is a sterile-filled vial or ampoule containing an injectable solution which comprises an effective amount of GLP-2 and at least one other peptide hormone selected from the group consisting of IGF-1, IGF-2 and GH dissolved in an aqueous vehicle.

According to the present invention, the GLP-2 in combination with at least one other peptide hormone selected from the group consisting of IGF-1, IGF-2 and GH is administered to treat patients who would benefit from enhanced upper gastrointestinal tract functioning. In one aspect, patient candidates are those who would benefit from proliferation of the upper gastrointestinal tract tissue. In addition, patient candidates are those who would benefit from increased upper gastrointestinal tract tissue function, whether as a result of increased tissue growth or not. The effects of combination therapy on these tissues, would clearly benefit those patients suffering from diseases or conditions marked by abnormalities. In general, patients who would benefit from increased upper gastrointestinal tract mass or regeneration and healing of preexistent normal mucosal epithelium are candidates for treatment with the invention. In addition, patients who would benefit from increased upper gastrointestinal tract tissue function, whether as a result of increased tissue growth or not, are candidates for treatment with the invention. For example, patients may be treated prior to or after a regimen of chemotherapy or radiotherapy, prior to, during, or following the onset of gastrointestinal inflammation, after a period of parenteral nutrition, after or during active gastrointestinal disease, or if the patient is a premature infant with insufficient maturation of the gastrointestinal tract, and/or inflammation of the GI tract as exemplified by the condition necrotizing enterocolitis.

Additional candidates for GLP-2 therapy include patients who are hospitalized, often in intensive care units, who are known to be at increased risk for upper GI tract inflammation and ulceration, or patients on drugs, such as NSAIDs and/or glucocorticoids, that increase the risk of upper GI tract inflammation/ulceration.

Numerous factors may be taken into consideration by a clinician when determining an optimal dosage for a given subject. Primary among these is the amount of peptide hormone normally produced by the body. Additional factors include the size of the patient, the age of the patient, the general condition of the patient, the particular disease being treated, the severity of the disease, the presence of other drugs in the patient, the *in vivo* activity of the peptide or peptide analog and the like. The trial dosages would be chosen after consideration of the results of animal studies and the clinical literature with respect to administration of peptide hormones and peptide hormone secretagogues. It will be appreciated by the person of ordinary skill in the art that information such as binding constants and Ki derived from *in vitro* binding competition assays may also be used in calculating dosages. It is expected that doses of the combination equivalent to about 0.1 mg/kg of GLP-2 and GLP-2 analogs twice daily, with approximately 2 mg/kg of IGF-1 twice daily and/or 1 mg/kg of GH once a day, co-administered over 10 days can generate very significant increases in the upper gastrointestinal tract. It is expected that much smaller doses, in the µg/kg range and perhaps into the ng/kg range, and perhaps shorter or longer duration or frequency of treatment, will also produce therapeutically useful results, *e.g.,* a statistically significant increase, particularly in small intestine mass.

A typical human dose of a GLP-2 peptide would be from about 10 µg/kg body weight/day to about 10 mg/kg/day, preferably from about 50 µg/kg/day to about 5 mg/kg/day, and most preferably about 100 µg/kg/day to 1 mg/kg/day. As the GLP-2 analogs of the invention can be up to 10 to even 100 times more potent than GLP-2, a typical dose of such a GLP-2 analog may be lower, for example, from about 100ng/kg body weight/day to 1mg/kg/day, preferably 1µg/kg/day to 500µg/kg/day, and even more preferably 1µg/kg/day to 100µg/kg/day.

For administration of GH to a mammal, particularly humans, a dose range of 0.02-2.5 mg/kg/day may be used; preferably, GH doses may range from about 0.1-2 mg/kg/day. For administration of IGF's, dosages may be in the range of 1 µg to 1 g/kg/day. IGF-1 is preferably administered to humans in the range of about 0.03-10 mg/kg/day, more preferably from about 0.1-4 mg/kg/day, and most preferably from around 0.5-1 mg/kg/day. Typical doses for IGF-2 are about 0.5-5 mg/kg/day. The dose required for analogues of IGF may be 20-50% less than naturally occurring IGF-1. Further, the dosage sizes and dosing regimen most appropriate for human use can be determined in properly designed clinical trials.

In another of its aspects, the invention provides for the treatment of patients as just identified using implanted intestinal cells that have been regenerated or stimulated to proliferate *in vitro* or *in vivo* prior to reimplantation or transplantation into a recipient. Conditioning of the cells *ex vivo* can be achieved simply by growing the cells or tissue to be transplanted in a medium that has been supplemented with a growth-promoting amount of the combinations and is otherwise appropriate for culturing of those cells. The cells can, after an appropriate conditioning period, then be implanted either directly into the patient or can be encapsulated using established cell encapsulation technology, and then implanted. Analogous procedures with other organs, such as the skin, are already fairly advanced clinically in human trials. For example, skin may be regenerated in vitro by taking skin cells from a donor, growing them up in tissue culture, and then transplanting back the expanded mass of skin back into a patient for therapeutic use (*e.g*., treatment of burns, ulcers, etc).

Further, the methods of the invention may be practiced using gene therapy. The recipient mammal's or patient's cells (which may be any type of cells but which are preferably fibroblasts or keratinocytes) can be engineered to express one or more of the subject growth factors, or combinations of GLP-2 with IGF-1 and/or IGF-2 and/or GH *in vitro*, followed by reimplantation, preferably in a capsule, *in vivo* for delivery of therapeutic amounts of these peptides. A variety of transfection techniques are currently available and used to transfer DNA *in vitro* into cells; including calcium phosphate-DNA precipitation, DEAE-Dextran transfection, electroporation, liposome-mediated DNA transfer or transduction with recombinant viral vectors. Such *ex vivo* treatment protocols have been proposed to transfer DNA into a variety of different cell types including epithelial cells (U.S. Patent 4,868,116; Morgan and Mulligan WO87/00201; Morgan et al., 1987, *Science* 237:1476-1479; Morgan and Mulligan, U.S. Patent No. 4,980,286); endothelial cells (WO89/05345); hepatocytes (WO89/07136; Wolff et al., 1987, *Proc. Natl. Acad. Sci. USA* 84:3344-3348; Ledley et al., 1987 *Proc. Natl. Acad. Sci.* 84:5335-5339; Wilson and Mulligan, WO89/07136; Wilson et al., 1990, *Proc. Natl. Acad. Sci.* 87:8437-8441); fibroblasts (Palmer et al., 1987, *Proc. Natl. Acad. Sci. USA* 84:1055-1059; Anson et al., 1987, *Mol. Biol. Med.* 4:11-20; Rosenberg et al., 1988, *Science* 242:1575-1578; Naughton & Naughton, U.S. Patent 4,963,489); lymphocytes (Anderson et al., U.S. Patent No. 5,399,346; Blaese, R.M., et al., 1995, *Science* 270:475-480); and hematopoietic stem cells (Lim, B., et al. 1989, *Proc. Natl. Acad. Sci. USA* 86:8892-8896; Anderson et al., U.S. Patent No. 5,399,346).

It is expected that gene therapy is a viable method of providing growth hormones for the practice of the invention. Specifically, one can conduct an experiment in which cells from a cell line which secretes all the glucagon peptides are implanted into a mouse. The implanted cells grow as a tumor which secretes GLP-2 and induces growth of the small intestine. Since GLP-2 is the only glucagon derived peptide known to markedly stimulate small intestine growth, the effect on small intestine growth observed in this experiment was most likely due to the secretion of GLP-2. Similarly, cells may be engineered to produce GLP-2 alone, and/or at least one peptide hormone selected from the group consisting of IGF-1, IGF-2 and GH and implanted into a mammal.

Alternatively, one may use gene therapy to transfect *in vivo* the recipient's cells. Formulations of nucleic acid for such *in vivo* methods may include: naked DNA; nucleic acid encapsulated into liposomes or liposomes combined with viral envelope receptor proteins (Nicolau et al., 1983, *Proc. Natl. Acad. Sci. USA* 80:1068); DNA coupled to a polylysine-glycoprotein carrier complex; and nucleic acid precipitants. Nucleic acid preparations may be introduced in *vivo* using any one of the techniques known in the art such as direct injection, electroporation, and particle bombardment. In addition, "gene guns" have been used for gene delivery into cells (Australian Patent No. 9068389).

A related approach might involve the use of modified gene therapy and viral vectors encoding the gene products for GLP-2 and at least one other peptide hormone selected from the group consisting of IGF-1, IGF-2 and GH. Such viral vectors may be used to infect remnant intestinal epithelium in patients with intestinal compromise, thereby effecting targeted local therapeutic delivery of these substances to the intestine *in vivo.* However, it is not necessary for the practice of the invention that all cells are transformed, or even that intestinal cells be transformed. Indeed, cells in any tissue which can secrete the chosen hormone into the circulatory system, for example muscle cells, may be used to endogenously produce hormones.

The sequence of such DNAs for the practice of gene therapy methods can readily be determined from the amino acid sequences of the selected GLP-2, IGF-1, IGF-2 and GH with the limitation that only forms containing genetically encoded amino acids can be produced in this manner. Various expression vectors, including viral vectors, suitable for introduction of genetic information into human cells, can be employed and will incorporate the DNA's encoding GLP-2, IGF-1, IGF-2 and GH under expression controls functional in the host cells. Once altered genetically, the engineered cells can then be implanted using procedures established in the art.

The invention having been described, the following examples are offered by way of illustration and not limitation.

### Example 1

In this experiment, the effect of GLP-2 on cell proliferation of the mucosal epithelium and muscle layer of the esophagus was examined.

Female CD1 mice (6 weeks of age, approx. 25g, Charles River) were housed in plastic bottom, wire lid cages and maintained on a 12 hr light/dark cycle and allowed chow and water ad libitum. The mice were weighed using a Mettler PJ300 scale and randomly allocated to either the control group (phosphate buffer saline injection ("PBS")) or the treatment group (human [Gly²]GLP-2 injection). Each control or treatment group consisted of 2 mice housed together in 1 cage.

The female CD1 mice were treated (subcutaneously) with either human [Gly²]GLP-2 2.5 µg or PBS alone, twice daily (8a.m. and 6 p.m.) for 2 days prior to the bromodeoxyuridine ("BrdU") injection (50ug/g body weight, in 0.5ml). The mice received a total of 5 injections of either human [Gly²]GLP-2 or saline over the previous 50 hours. Mice were fasted 21 hours before sacrifice. The mice were sacrificed 2 hours after receiving the BrdU injection. Upon sacrifice, sections of distal esophagus (the last 2 cm of esophagus adjacent to the gastroduodenal junction) were taken for histological analysis.

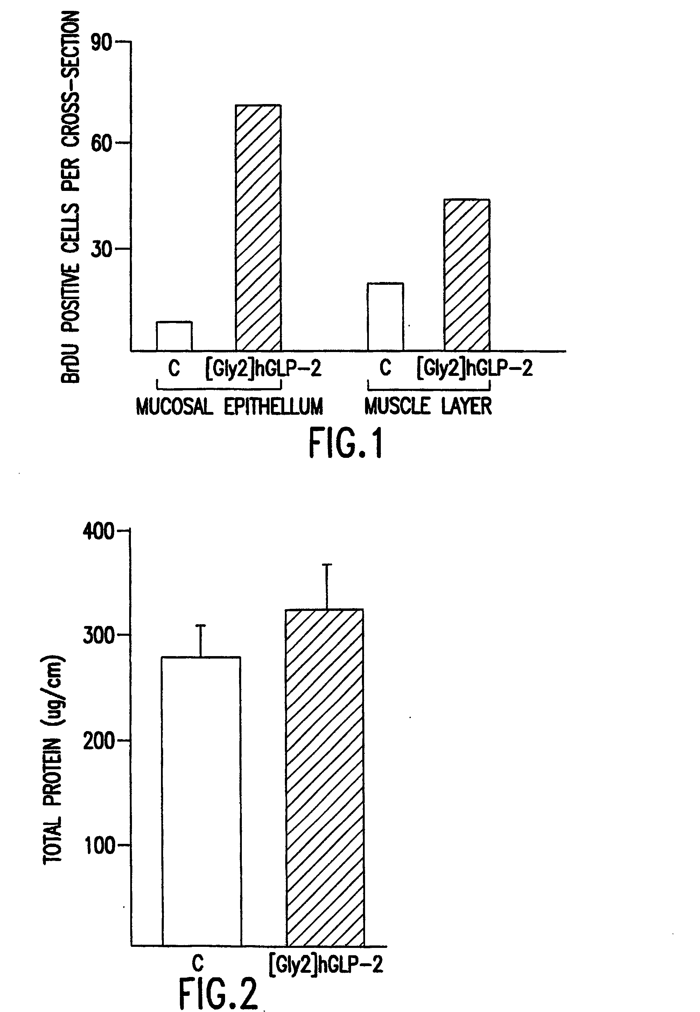

The esophageal tissues were fixed in 10% buffered formalin for over 24 hours and then placed in 70% alcohol prior to processing. Tissues were processed and embedded in paraffin using standard techniques. Four to six micron cross-sections were cut and stained with hematoxylin and eosin and a second set of slides were stained for BrdU-immunopositive cells. Intestinal micrometry was performed using the Leica Q500MC Image Analysis System. Results are shown in Figure 1.

The results of this experiment clearly show that administration of human [Gly²]GLP-2 over a 50 hr period produces significant increases in cellular proliferation of the mucosal epithelium and muscle layer of the esophagus as assessed by the number of BrDU-positive cells.

### Example 2

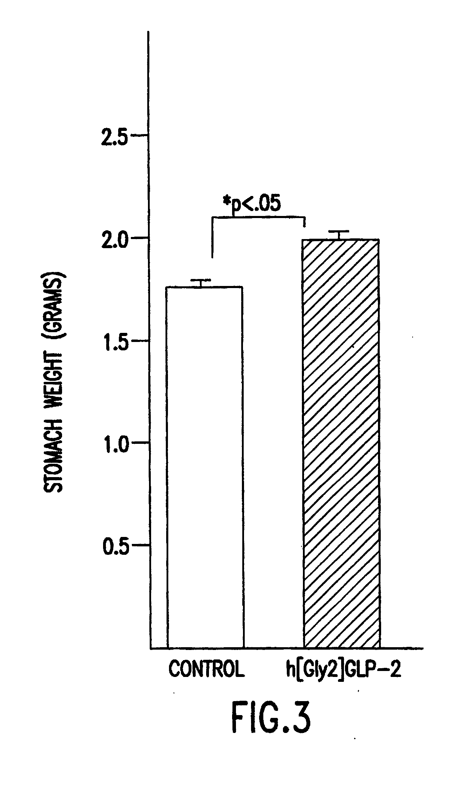

In this experiment, the effect of human [Gly²] GLP-2 on protein synthesis in the mouse esophagus was examined. Female CD1 mice (6 weeks old) were housed and fed as described in Example 1. Two groups of mice (n=5 per group) were treated twice daily (subcutaneously) with either 0.5 ml PBS alone (control) or 2.5 ug in 0.5 ml PBS for 10 days. Following treatment, mice were anesthetized with CO₂ and sacrificed for analysis. One (1) cm segments of esophagus tissue, between 2-4 cm proximal to the gastroduodenal junction, were harvested for analysis of total cellular protein. The 1 cm esophageal segments were placed in 1 ml of PBS and homogenized using a Polytron. Aliquots of the homogenate were taken for protein determination of protein content using the modified Bradford assay (BIORAD). Results are shown in Figure 2.

The protein content assay results show an increase in protein synthesis in the esophagus tissue following treatment with human [Gly²]GLP-2. These results are consistent with the cellular action of human [Gly²]GLP-2 on its target receptor in this tissue.

### Example 3

In this experiment, the effect of GLP-2 on the stomach mass was examined. Female CD1 mice (6 weeks old) were treated with human [Gly²]GLP-2 by oral administration in drinking water. The drinking water contained 4.3µg/ml of human [Gly²]GLP-2 and was prepared fresh daily. Each mouse received approximately 100µg of human [Gly²]GLP-2 per day. Following 12 days of treatment the mice were fasted for 24 hours, anesthetized with CO₂ and sacrificed. The stomach of each mouse was removed and weighed. Result are shown in Figure 3.

The weights of the stomachs significantly increase with treatment of human [Gly²] GLP-2 (p<0.05) as compared to control mice who received normal drinking water.

### Example 4

In this experiment, the prophylactic and therepeutic effect of GLP-2 on indomethacin induced mucosal epithelial damage was studied.

Nonsteroidal anti-inflammatory drugs (NSAIDs) are among the most commonly consumed medications in North America. Therapeutically, they have analgesic, anti-inflammatory, antipyretic, and antiplatelet properties. Gastrointestinal toxicity remains a major cause of morbidity despite attempts at prophylaxis using agents targeted at gastric acid suppression or via the administration of exogenous prostaglandins. Estimates of the incidence of NSAID-induced gastropathy, ranging from asymptomatic gastric erosions to life-threatening intestinal bleeds, varies between 15% and 20% in chronic NSAID users. The precise incidence of small intestinal damage (NSAID-induced enteropathy) remains unknown.

NSAIDs block the access of arachidonic acid to the binding site of cyclo-oxygenase (prostaglandin synthase) preventing the formation of prostaglandins and thromboxane. Suppression of prostaglandin synthesis, however, does not appear to be a major contributor to NSAID-induced small intestinal mucosal injury. Rather, alterations in epithelial permeability, increased luminal bacterial load, and bile contribute to epithelial injury. In rodents administered NSAIDs, in addition to gastropathy, small intestinal and colonic transmural inflammation and ulceration are noted, hence NSAID administration may be used to generate an animal model for inflammatory bowel disease.

GLP-2 administration to mice and rats stimulates mucosal growth in both the small and large intestine via activation of crypt cell proliferation and inhibition of enterocyte apoptosis. To address the possibility that GLP-2 may be useful for the therapeutic and/or prophylactic treatment of mucosal epithelial damage, we have studied the use of a novel human GLP-2 analog, h[Gly²]-GLP-2, in mice with experimental indomethacin induced gastroenterocolitis.

### Materials and Methods

### Animals

Six to eight week old female CD1 mice (approximately 22-28 g, Charles River) were housed in plastic bottom, wire lid cages and maintained on a 12 hr light /dark cycle and allowed chow and water ad libitum throughout the study. The day prior to commencing the experiment, mice were weighed using a Mettler PJ300 scale and randomly allocated to a treatment group. Each treatment group consisted of 5 mice housed together. Subcutaneous injections of PBS or h[Gly²]-GLP-2 peptide were given in the right hind quarter twice a day at 9 am and 7 pm. Injections started 4 days before indomethacin administration. Mice were fasted the night prior to receiving the first dose of indomethacin.

Mice were sacrificed between 9 am and 5 pm. Weight measurements were taken of the stomach, small intestine, and large intestine. Small and large bowel length were also measured. Blood was taken via cardiac puncture and the plasma isolated by centrifugation at 10,000 g for 10 minutes. Plasma was frozen at -70° C for future analysis of GLP-2 levels. Tissues were removed for histological analysis, protein, wet and dry weight, RNA, and myeloperoxidase assay.

### GLP-2 Administration

50.4 mg h[Gly²]GLP-2 (ALX-0600) obtained from Allelix on August 1, 1997 dissolved in sterile H₂O; 5N NaOH was used to pH the solution to a final pH 7. This batch of peptide was used for all experiments. Aliquots of 0.2, 0.5, 1.0, 2.0 mg/ml were frozen at -80° C. 600 µg of h[Gly²]-GLP-2 in 1 ml solution was aliquoted into 399 ml phosphate buffer saline (PBS - 137 mM NaCl, 2.7 mM KCl, 4.3 mM Na2HPO4·7H2O, 1.4 mM KH2PO4, pH 7.3) to obtain a final concentration of 1.5 ug/mL. 14 ml aliquots were stored at -80° C and thawed in a 37° C waterbath prior to injection. The injection volume was 0.5 ml per injection using 1/2 cc U-100 Insulin Syringes (Becton Dickinson and Company, NJ). Animals injected with PBS received the same PBS solution used to dilute the h[Gly²]-GLP-2 peptide via an identical route of administration.

### Indomethacin Administration

Indomethacin(1-[p-chlorobenzoyl]-5-methoxy-2-methylindole-3-acetic acic) (Sigma Chemical Co., St Louis MO, lot 74H1212), stock solution was prepared and aliquoted 1 hr before the first injection. The mice were weighed, and then fasted overnight, and given food one hour prior to the first indomethacin injection. Mice were not fasted for the second indomethacin injection. Indomethacin (14.5 mg) was weighed out using a Mettler AE166 scale and dissolved in 1 mL anhydrous ethyl alcohol. The solution was warmed at 37° C for 5 minutes and thoroughly vortexed every minute until fully dissolved. 998.1 uL of stock indomethacin solution was diluted in 41 ml 5% NaHCO3 pH 7.3. Animals received 0.5 ml injections at 10:00 AM O.D. over two days. Indomethacin used in the second injection was stored at -20° C overnight and allowed to thaw at room temperature prior to injection. Mice were injected at the same time (10:00 AM) on both days approximately 1 hr after receiving h[Gly²]-GLP-2. Animals not receiving indomethacin were administered vehicle alone (250 µl anhydrous ethyl alcohol in 10.5 mL NaHCO3 pH 7.3 - a final injection volume of 0.5 ml was given daily over 2 days). Animals were sacrificed in a CO₂ chamber 14 days following the administration of either PBS or h[Gly²]-GLP-2.

### Results

Pilot experiments were performed to determine an indomethacin dose that reproducibly produced ∼ 50% mortality in mice. For all groups receiving indomethacin treatment in this study, a dose of indomethacin 7 mg/kg administered *s.c.* bid for two days following overnight fasting was administered to 7-8 week old female CD1 mice. This dose consistently resulted in 50% mortality in saline-treated control animals. In each treatment group, subcutaneous injections of PBS or h[Gly²]-GLP-2, .75 µg were administered twice daily to the animals receiving indomethacin and to the negative controls.

Treatment group I was used to characterize the type and extent of bowel injury produced by indomethacin (dose 7 mg/kg). Animals were pretreated with saline or h[Gly²]-GLP-2 for 4 days, followed by an additional 2 days of either saline or h[Gly²]-GLP-2 and indomethacin, then sacrificed two days following the initial indomethacin dose.

Treatment group II was used to examine the effect of four days of pretreatment with either h[Gly²]-GLP-2 or saline followed by ten days of additional treatment with saline or h[Gly²]-GLP-2.

Treatment group III was used to examine effects on mice treated with saline or h[Gly²]-GLP-2 for 4 days prior and 2 days concurrent with indomethacin administration.

Treatment groups IV and V of the study involved mice receiving saline or h[Gly²]-GLP-2 commencing concurrently (IV) or the day following (V) indomethacin administration and continued until the animals were sacrificed at the end of the 10 day experimental period.

All h[Gly²]-GLP-2-treated mice in groups II-V exhibited a marked increase in survival, ranging from 75-95%, in contrast with survival in saline-treated controls that ranged from 45-50%. This difference was highly statistically significant (P<.05-.01). Experimental endpoints included small and large bowel weights, histology, disease activity scores, mucosal enzyme activity, small and large bowel epithelial-specific gene expression, markers of the inflammatory response such as expression of cytokines and myeloperoxidase and GLP-2 content. The majority of parameters assessed, especially markers of epithelial damage/inflammation, were significantly improved in the h[Gly²]-GLP-2-treated mice.

## Claims

1. Use of a GLP-2 receptor agonist in the manufacture of a medicament for enhancing the functioning of the upper gastrointestinal tract including the esophagus and the stomach, wherein the medicament is intended to be delivered to the upper gastrointestinal tract.

2. Use according to claim 1, wherein the GLP-2 receptor agonist is GLP-2 or a peptidic analog of GLP-2 , which is intended to be delivered to the esophagus.

3. Use according to claim 1, wherein the GLP-2 receptor agonist is GLP-2 or a peptidic analog of GLP-2, which is intended to be delivered to the stomach.

4. Use of a peptidic analog of GLP-2, in the manufacture of a medicament, to proliferate the tissue of the upper gastrointestinal tract, wherein the medicament is intended to be delivered to the upper gastrointestinal tract.

5. Use according to claims 1 or 4, wherein the subject is suffering from an inflammatory condition involving the upper gastrointestinal tract.

6. Use according to claim 5, wherein the inflammatory condition involving the upper gastrointestinal tract is an inflammatory condition involving the esophagus.

7. Use according to claim 6, wherein the inflammatory condition involving the esophagus is selected from the group consisting of inflammation-vasculitis, post-infection inflammation, infiltrative disorder, infectious esophagitis, non-infectious esophagitis, sarcoidosis, Chrohn's disease, Behcet's disease, graft-versus-host disease, acid reflux, bile reflux, drug-induced injury, chemical injury, radiation, myositis or collagen vascular diseases.

8. Use according to claim 5, wherein the inflammatory condition involving the upper gastrointestinal tract is an inflammatory condition involving the stomach.

9. Use according to claim 8, wherein the condition involving the stomach is selected from the group consisting of hemorrhagic and erosive gastritis, *Helicobacter pylori* gastritis, chemical gastritis, metaplastic atrophic gastritis, postantrectomy atrophic gastritis, eosinophilic gastritis, infectious gastritis, Crohn's disease, sarcoidosis, isolated granulomatous gastritis, lymphocytic gastritis, and Ménétriere's disease.

10. Use according to claim 4, wherein the subject has undergone partial or subtotal resection of the upper gastrointestinal tract.

11. Use according to claim 10, wherein the partial or total resection of the upper gastrointestinal tract involves the esophagus or the stomach.

12. Use according to claims 1-11, wherein the subject is a human.

13. Use according to claim 12, wherein the analog of GLP-2 has enhanced upper gastrointestinal tract cell proliferating activity relative to native rat GLP-2.

14. Use according to claim 13, wherein the analog of GLP-2 is human GLP-2.

15. Use according to claim 14, wherein the analog of GLP-2 is intended to be delivered to the upper gastrointestinal tract by oral, subcutaneous, or intravenous administration.

16. A method to identify peptides useful to treat inflammatory conditions involving the upper gastrointestinal tract, comprising the steps of:
a) obtaining an analog of a vertebrate GLP-2 peptide, the analog having at least one amino acid substitution, deletion, addition, or an amino acid with a blocking group;
b) inducing an inflammatory condition involving the upper gastrointestinal tract in a test animal; and
c) determining the effect of the analog on the health status or mortality of the test animal having an induced inflammatory condition of the upper gastrointestinal tract compared with control animals not receiving the peptide or determining the effect of the analog on the weight of the upper gastrointestinal tract of test animals compared to control animals not receiving peptide.

17. A method to identify peptides useful to prevent or ameliorate inflammatory conditions involving the upper gastrointestinal tract, comprising the steps of:
a) obtaining an analog of a vertebrate GLP-2 peptide, the analog having at least one amino acid substitution, deletion, addition, or an amino acid with a blocking group;
b) inducing an inflammatory condition involving the upper gastrointestinal tract in a test animal having an induced inflammatory condition of the upper gastrointestinal tract compared with control animals; and
c) determining the effect of the analog on the health status or mortality of the test animal compared with control animals not receiving the peptide or determining the effect of the analog on the weight of the upper gastrointestinal tract of test animals compared to control animals not receiving peptide.

18. A method useful to identify peptides capable of proliferating tissue of the upper gastrointestinal tract, comprising the steps of:
a) obtaining an analog of a vertebrate GLP-2 peptide, the analog having at least one amino acid substitution, deletion, addition, or an amino acid with a blocking group;
b) delivering the analog to the upper gastrointestinal tract of the test animal using a regimen capable of eliciting proliferation of the upper gastrointestinal tract when utilized for native GLP-2; and
c) assessing the increase in the mass, length, or total protein content of the upper gastrointestinal tract or representative portion after completion of the treatment regimen.

19. Use of GLP-2 or a GLP-2 analog in the manufacture of a medicament for inhibiting the onset and/or ameliorating the development of an inflammatory condition involving inflammation of the upper gastrointestinal tract in a subject at risk of developing such a condition.

20. Use of a GLP-2 receptor agonist and at least one peptide hormone in the in the manufacture of a medicament for promoting the growth of upper gastrointestinal tract tissue in a mammal such that the serum levels of said receptor agonist and peptide hormone are elevated, and wherein the GLP-2 receptor agonist is GLP-2 or an analog of GLP-2 and the at least one other peptide hormone is selected from the group consisting of IGF-1, analogs of IGF-1, IGF-2, analogs of IGF-2, GH, and analogs of GH.

21. Use according to claim 20 comprising co-administering to a mammal an effective amount of GLP-2, or an analog of GLP-2, and an effective amount of at least one other peptide hormone selected from the group consisting of IGF-1, analogs of IGF-1, IGF-2, analogs of IGF-2, GH, and analogs of GH.

22. Use according to claim 20, wherein the cells have been genetically engineered *ex vivo* to produce GLP-2 and/or the other peptide hormone, and are for implantation into the mammal.

23. Use according to claim 20 wherein GLP-2 and/or the other peptide hormone is to be produced in the mammal by in vivo engineered cells.

24. Use according to claim 20 wherein the peptide hormone is IGF-1 or LRIGF-1.

25. Use according to claim 20 wherein the peptide hormone is IGF-2.

26. Use according to claim 20 wherein the peptide hormone is GH.

27. Use according to claim 20 for promoting the growth of the esophagus.

28. Use according to claim 20 for promoting the growth of the stomach.

29. Use of a GLP-2 receptor agonist and at least one peptide hormone in the manufacture of a medicament to enhance the functioning of the upper gastrointestinal tract such that the serum levels of said receptor agonist and peptide hormone are elevated and wherein the GLP-2 receptor agonist is GLP-2, or an analog of GLP-2, and the at least one other peptide hormone is selected from the group consisting of IGF-1, analogs of IGF-1, IGF-2, analogs of IGF-2, GH, analogs of GH, EGF, analogs of EGF, HGF, analogs of HGF, KGF, and analogs of KGF.

30. Use according to claim 29 for preserving, restoring or maintaining upper gastrointestinal tract function:
after a regimen of chemotherapy or radiotherapy;
after a period of parenteral nutrition;
after gastrointestinal disease; or
wherein said patient is a premature infant.

31. Use according to claim 29, wherein the subject is suffering from an inflammatory condition involving the upper gastrointestinal tract.

32. Use according to claim 31, wherein the inflammatory condition involving the upper gastrointestinal tract is an inflammatory condition involving the esophagus.

33. Use according to claim 32, wherein the inflammatory condition involving the esophagus is selected from the group consisting of inflammation-vasculitis, post-infection inflammation, infiltrative disorder, infectious esophagitis, non-infectious esophagitis, sarcoidosis, Crohn's disease, Behcet's disease, graft-versus-host disease, acid reflux, bile reflux, drug-induced injury, chemical injury, radiation, myositis or collagen vascular diseases.

34. Use according to claim 31, wherein the inflammatory condition involving the upper gastrointestinal tract is an inflammatory condition involving the stomach.

35. Use according to claim 34, wherein the inflammatory condition involving the stomach is selected from the group consisting of hemorrhagic and erosive gastritis, *Helicobacter pylori* gastritis, chemical gastritis, metaplastic atrophic gastritis, postantrectomy atrophic gastritis, eosinophilic gastritis, infectious gastritis, Crohn's disease, sarcoidosis, isolated granulomatous gastritis, lymphocytic gastritis, gastric ulceration, and Ménétriere's disease.

36. Use according to claim 29, wherein the subject has undergone partial or subtotal resection of the upper gastrointestinal tract.

37. Use according to claim 36, wherein the partial or total resection of the upper gastrointestinal tract involves the esophagus or the stomach.

38. Use according to claims 29-37, wherein the subject is a human.

39. Use according to claim 38, wherein the analog of GLP-2 has enhanced upper gastrointestinal tract cell proliferating activity relative to native rat GLP-2.

40. Use according to claim 39, wherein the analog of GLP-2 is human GLP-2.

41. Use according to claim 40, wherein the analog of GLP-2 is intended to be delivered to the upper gastrointestinal tract by oral, subcutaneous, or intravenous administration.

42. A kit comprising GLP-2 or an analog thereof and at least one other peptide hormone selected from the group consisting of IGF-1, IGF-2, GH, EGF, HGF and KGF in a therapeutically effective unit dose or multi-dose amount.

43. A method for promoting the growth of upper gastrointestinal tract tissue or cells which comprises the step of culturing said tissue or cells in a culturing medium containing a growth promoting combination of both GLP-2, or an analog of GLP-2, and at least one other peptide hormone selected from the group consisting of IGF-1, analogs of IGF-1, IGF-2, analogs of IGF-2, GH, analogs of GH, EGF, analogs of EGF, HGF, analogs of HGF, KGF, and analogs of KGF.

44. Use of a GLP-2 receptor agonist and at least one peptide hormone for the manufacture of cultured upper gastrointestinal tract tissue as obtained in claim 43 for use for restoring the upper gastrointestinal tract of a patient wherein the tissue is for implanting in the patient.

45. A method for determining the activity of a hormone when used in combination with GLP-2, said method comprising the steps of:
(a) coadministering the hormone with an amount of GLP-2, or a GLP-2 analog, to a test mammal,
(b) assessing the subsequent growth of upper gastrointestinal tract tissue in the test mammal, and
(c) determining whether the growth of upper gastrointestinal tract tissue in the test mammal is enhanced relative to control mammals treated with GLP-2 alone.

46. Use of GLP-2 or an analog of GLP-2, and at least one other agent effective for treating peptidic ulcers in the manufacture of a medicament for treating a subject having peptidic ulcers wherein the medicament is intended to be delivered to the upper gastrointestinal tract of the subject and the at least one other agent effective for treating peptidic ulcers is selected from the group consisting of: agents that act to block acid secretion from the stomach, agents that act to form a protective barrier in the stomach, or bismuth containing compounds, in an effective therapeutic amount.

47. Use according to claim 46 wherein the agents that act to block acid secretion from the stomach include H₂ receptor antagonists, proton pump inhibitors and protaglandins.

48. Use according to claim 46 wherein the agents that act to form a protective barrier in the stomach includes sucralfat.

49. Use according to any one of claims 1-12, 19-38 and 43-48, wherein the GLP-2 is a vertebrate GLP-2.

50. Use according to any one of claims 1-12, 19-38 and 43-48, wherein the GLP-2 is a mammalian GLP-2.

51. Use according to claim 50, wherein the GLP-2 is human GLP-2.

52. Use according to any one of claims 1-12, 19-38, and 43-48, wherein the GLP-2 is an analog of GLP-2 which incorporates at least one amino acid addition, substitution or deletion.

53. Use according to claim 52, wherein the GLP-2 analog incorporates an amino acid substitution.

54. Use according to claim 53, wherein the GLP-2 analog is human or rat GLP-2 analog and incorporates an amino acid substitution at position 2 which confers on said analog resistance to cleavage by DPP-IV enzyme.

55. Use according to claim 54, wherein the GLP-2 analog is human GLP-2 analog and incorporates a glycine substitution at position 2.

## Patentansprüche

1. Verwendung eines GLP-2-Rezeptor-Agonisten bei der Herstellung eines Medikaments zur Verbesserung der Funktion des oberen Magen-Darm-Trakts einschließlich der Speiseröhre und des Magens, bei der das Medikament für eine Abgabe an den oberen Magen-Darm-Trakt vorgesehen ist.

2. Verwendung nach Anspruch 1, bei welcher der GLP-2-Rezeptor-Agonist GLP-2 oder ein peptidisches Analog von GLP-2 ist, das für eine Abgabe an die Speiseröhre vorgesehen ist.

3. Verwendung nach Anspruch 1, bei welcher der GLP-2-Rezeptor-Agonist GLP-2 oder ein peptidisches Analog von GLP-2 ist, das für eine Abgabe an den Magen vorgesehen ist.

4. Verwendung eines peptidischen Analogs von GLP-2 bei der Herstellung eines Medikaments, um das Gewebe des oberen Magen-Darm-Trakts zur Proliferation zu bringen, bei der das Medikament für eine Abgabe an den oberen Magen-Darm-Trakt vorgesehen ist.

5. Verwendung nach Anspruch 1 oder 4, bei welcher der Zielorganismus an einem Entzündungszustand leidet, der den oberen Magen-Darm-Trakt betrifft.

6. Verwendung nach Anspruch 5, bei welcher der den oberen Magen-Darm-Trakt betreffende Entzündungszustand ein Entzündungszustand ist, der die Speiseröhre betrifft.

7. Verwendung nach Anspruch 6, bei welcher der die Speiseröhre betreffende Entzündungszustand aus der Gruppe ausgewählt wird, die aus folgenden besteht: Entzündungsvaskulitis, postinfektiöse Entzündung, Infiltrationserkrankung, infektiöse Ösophagitis, nichtinfektiöse Ösophagitis, Sarkoidose, Morbus Crohn, Behcet-Krankheit, Graft-versus-host-Krankheit, Säurerückfluß, Gallerückfluß, medikamenteninduziertes Trauma, chemisches Trauma, Strahlungstrauma, Myositis oder Kollagenosen,

8. Verwendung nach Anspruch 5, bei welcher der den oberen Magen-Darm-Trakt betreffende Entzündungszustand ein Entzündungszustand ist, der den Magen betrifft.

9. Verwendung nach Anspruch 8, bei welcher der den Magen betreffende Zustand aus der Gruppe ausgewählt wird, die aus folgenden besteht: hämorrhagische und erosive Gastritis, *Helicobacter-pylori*-Gastritis, chemische Gastritis, metaplastische atrophische Gastritis, atrophische Gastritis nach Antrektomie, eosinophile Gastritis, infektiöse Gastritis, Morbus Crohn, Sarkoidose, isolierte granulomatöse Gastritis, lymphozytische Gastritis und Morbus Ménétrier.

10. Verwendung nach Anspruch 4, bei der sich der Zielorganismus einer partiellen oder subtotalen Resektion des oberen Magen-Darm-Trakts unterzogen hat.

11. Verwendung nach Anspruch 10, bei der die partielle oder subtotale Resektion des oberen Magen-Darm-Trakts die Speiseröhre oder den Magen betrifft.

12. Verwendung nach Anspruch 1 bis 11, bei welcher der Zielorganismus ein Mensch ist.

13. Verwendung nach Anspruch 12, bei der das Analog von GLP-2 die Zellproliferationsaktivität des oberen Magen-Darm-Trakts im Verhältnis zu nativem Ratten-GLP-2 gesteigert hat.

14. Verwendung nach Anspruch 13, bei der das Analog von GLP-2 ein Analog von Human-GLP-2 ist.

15. Verwendung nach Anspruch 14, bei der das Analog von GLP-2 dafür vorgesehen ist, durch orale, subkutane oder intravenöse Verabreichung an den oberen Magen-Darm-Trakt abgegeben zu werden.

16. Verfahren zum Identifizieren von Peptiden, die zur Behandlung von Entzündungszuständen verwendet werden können, die den oberen Magen-Darm-Trakt betreffen, das die folgenden Schritte umfaßt:
a) Gewinnen eines Analogs eines Wirbeltier-GLP-2-Peptids, wobei das Analog wenigstens einen Aminosäureaustausch, -verlust, -zusatz oder eine Aminosäure mit einer blockierenden Gruppe hat,
b) Induzieren eines den oberen Magen-Darm-Trakt betreffenden Entzündungszustands bei einem Versuchstier und
c) Bestimmen der Wirkung des Analogs auf den Gesundheitszustand oder die Mortalität des Versuchstiers mit einem den oberen Magen-Darm-Trakt betreffenden induzierten Entzündungszustand im Vergleich mit Kontrolltieren, die das Peptid nicht erhalten, oder Bestimmen der Wirkung des Analogs auf das Gewicht des oberen Magen-Darm-Trakts von Versuchstieren im Vergleich mit Kontrolltieren, die das Peptid nicht erhalten.

17. Verfahren zum Identifizieren von Peptiden, die zum Verhindern oder Lindern von Entzündungszuständen verwendet werden können, die den oberen Magen-Darm-Trakt betreffen, das die folgenden Schritte umfaßt:
a) Gewinnen eines Analogs eines Wirbeltier-GLP-2-Peptids, wobei das Analog wenigstens einen Aminosäureaustausch, -verlust, -zusatz oder eine Aminosäure mit einer blockierenden Gruppe hat,
b) Induzieren eines den oberen Magen-Darm-Trakt betreffenden Entzündungszustands bei einem Versuchstier mit einem induzierten Entzündungszustand des oberen Magen-Darm-Trakts im Vergleich mit Kontrolltieren und
c) Bestimmen der Wirkung des Analogs auf den Gesundheitszustand oder die Mortalität des Versuchstiers im Vergleich mit Kontrolltieren, die das Peptid nicht erhalten, oder Bestimmen der Wirkung des Analogs auf das Gewicht des oberen Magen-Darm-Trakts von Versuchstieren im Vergleich mit Kontrolltieren, die das Peptid nicht erhalten.

18. Verfahren zum Identifizieren von Peptiden, die in der Lage sind, das Gewebe des oberen Magen-Darm-Trakts zur Proliferation zu bringen, das die folgenden Schritte umfaßt:
a) Gewinnen eines Analogs eines Wirbeltier-GLP-2-Peptids; wobei das Analog wenigstens einen Aminosäureaustausch, -verlust, -zusatz oder eine Aminosäure mit einer blockierenden Gruppe hat,
b) Abgeben des Analogs an den oberen Magen-Darm-Trakt des Versuchstiers unter Verwendung eines Regimes, das in der Lage ist, eine Proliferation des oberen Magen-Darm-Trakts hervorzurufen, wenn es für natives GLP-2 eingesetzt wird, und
c) Abschätzen der Zunahme bei der Masse, der Länge oder dem Gesamtproteingehalt des oberen Magen-Darm-Trakts oder eines repräsentativen Abschnitts nach dem Abschluß des Behandlungsregimes.

19. Verwendung von GLP-2 oder eines GLP-2-Analogs bei der Herstellung eines Medikaments zur Hemmung des Ausbruchs und/oder zur Linderung der Entwicklung eines Entzündungszustands, der eine Entzündung des oberen Magen-Darm-Trakts einschließt, bei einem Zielorganismus mit dem Risiko, einen solchen Zustand zu entwickeln.

20. Verwendung eines GLP-2-Rezeptor-Agonisten und wenigstens eines Peptidhormons bei der Herstellung eines Medikaments zur Förderung des Wachstums von Gewebe des oberen Magen-Darm-Trakts bei einem Säugetier derart, daß die Serumwerte des Rezeptor-Agonisten und des Peptidhormons erhöht werden, und bei welcher der GLP-2-Rezeptor-Agonist GLP-2 oder ein Analog von GLP-2 ist und das wenigstens eine andere Peptidhormon aus der Gruppe ausgewählt wird, die aus IGF-1, Analogen° von IGF-1, IGF-2, Analogen von IGF-2, GH, Analogen von GH, EGF, Analogen von EGF, HGF, Analogen von HGF, KGF und Analogen von KGF besteht.

21. Verwendung nach Anspruch 20, die umfaßt, einem Säugetier gemeinsam eine wirksame Menge GLP-2 oder eines Analogs von GLP-2 und eine wirksame Menge wenigstens eines anderen Peptidhormons zu verabreichen, ausgewählt aus der Gruppe, die aus IGF-1, Analogen von IGF-1, IGF-2, Analogen von IGF-2, GH und Analogen von GH besteht.

22. Verwendung nach Anspruch 20, bei der die Zellen ex vivo gentechnisch verändert worden sind, um GLP-2 und/oder das andere Peptidhormon zu erzeugen, und zum Implantieren in das Säugetier vorgesehen sind.

23. Verwendung nach Anspruch 20, bei der das GLP-2 und/oder das andere peptidhormon in dem Säugetier durch in vivo veränderte Zellen erzeugt werden soll.

24. Verwendung nach Anspruch 20, bei der das Peptidhormon IGF-1 oder LRIGF-1 ist.

25. Verwendung nach Anspruch 20, bei der das Peptidhormon IGF-2 ist.

26. Verwendung nach Anspruch 20, bei der das Peptidhormon GH ist.

27. Verwendung nach Anspruch 20 zum Fördern des Wachstums der Speiseröhre.

28. Verwendung nach Anspruch 20 zum Fördern des Wachstums des Magens.

29. Verwendung eines GLP-2-Rezeptor-Agonisten und wenigstens eines Peptidhormons bei der Herstellung eines Medikaments, um die Funktion des oberen Magen-Darm-Trakts zu verbessern derart, daß die Serumwerte des Rezeptor-Agonisten und des Peptidhormons erhöht werden, und bei welcher der GLP-2-Rezeptor-Agonist GLP-2 oder ein Analog von GLP-2 ist und das wenigstens eine Peptidhormon aus der Gruppe ausgewählt wird, die aus IGF-1, Analogen von IGF-1, IGF-2, Analogen von IGF-2, GH, Analogen von GH, EGF, Analogen von EGF, HGF, Analogen von HGF, KGF und Analogen von KGF besteht.

30. Verwendung nach Anspruch 29 zum Erhalten, Wiederherstellen oder Aufrechterhalten der Funktion des oberen Magen-Darm-Trakts
nach einem Chemotherapie- oder Strahlentherapieregime,
nach einem Zeitraum parenteraler Ernährung,
nach einer Magen-Darm-Erkrankung oder
bei welcher der Patient ein Frühgeborenes ist.

31. Verwendung nach Anspruch 29, bei welcher der Zielorganismus an einem Entzündungszustand leidet, der den oberen Magen-Darm-Trakt betrifft.

32. Verwendung nach Anspruch 31, bei welcher der den oberen Magen-Darm-Trakt betreffende Entzündungszustand ein Entzündungszustand ist, der die Speiseröhre betrifft.

33. Verwendung nach Anspruch 32, bei welcher der die Speiseröhre betreffende Entzündungszustand aus der Gruppe ausgewählt wird, die aus folgenden besteht: Entzündungsvaskulitis, postinfektiöse Entzündung, Infiltrationserkrankung, infektiöse Ösophagitis, nichtinfektiöse Ösophagitis, Sarkoidose, Morbus Crohn, Behcet-Krankheit, Graft-versus-host-Krankheit, Säurerückfluß, Gallerückfluß, medikamenteninduziertes Trauma, chemisches Trauma, Strahlungstrauma, Myositis oder Kollagenosen.

34. Verwendung nach Anspruch 31, bei welcher der den oberen Magen-Darm-Trakt betreffende Entzündungszustand ein Entzündungszustand ist, der den Magen betrifft.

35. Verwendung nach Anspruch 34, bei welcher der den Magen betreffende Entzündungszustand aus der Gruppe ausgewählt wird, die aus folgenden besteht: hämorrhagische und erosive Gastritis, *Helicobacter-pylori*-Gastritis, chemische Gastritis, metaplastische atrophische Gastritis, atrophische Gastritis nach Antrektomie, eosinophile Gastritis, infektiöse Gastritis, Morbus Crohn, Sarkoidose, isolierte granulomatöse Gastritis, lymphozytische Gastritis, Magengeschwür und Morbus Ménétrier.

36. Verwendung nach Anspruch 29, bei der sich der Zielorganismus einer partiellen oder subtotalen Resektion des oberen Magen-Darm-Trakts unterzogen hat.

37. Verwendung nach Anspruch 36, bei der die partielle oder subtotale Resektion des oberen Magen-Darm-Trakts die Speiseröhre oder den Magen betrifft.

38. Verwendung nach Anspruch 29 bis 37, bei welcher der Zielorganismus ein Mensch ist.

39. Verwendung nach Anspruch 38, bei der das Analog von GLP-2 die Zellproliferationsaktivität des oberen Magen-Darm-Trakts im Verhältnis zu nativem Ratten-GLP-2 gesteigert hat.

40. Verwendung nach Anspruch 39, bei der das Analog von GLP-2 ein Analog von Human-GLP-2 ist.

41. Verwendung nach Anspruch 40, bei der das Analog von GLP-2 dafür vorgesehen ist, durch orale, subkutane oder intravenöse Verabreichung an den oberen Magen-Darm-Trakt abgegeben zu werden.

42. Satz, der in einer therapeutisch wirksamen Einzeldosis- oder Mehrfachdosismenge GLP-2 oder ein Analog desselben und wenigstens ein anderes Peptidhormon umfaßt. ausgewählt aus einer Gruppe, die aus IGF-1, IGF-2, GH, EGF, HGF und KGF besteht.

43. Verfahren zum Fördern des Wachstums von Gewebe oder Zellen des oberen Magen-Darm-Trakts, das den Schritt umfaßt, das Gewebe oder die Zellen in einem Kulturmedium zu kultivieren, das eine wachstumsfördernde Kombination sowohl von GLP-2 oder einem Analog von GLP-2 als auch wenigstens einem anderen Peptidhormon enthält, ausgewählt aus der Gruppe, die aus IGF-1, Analogen von IGF-1, IGF-2, Analogen von IGF-2, GH, Analogen von GH, EGF, Analogen von EGF, HGF, Analogen von HGF, KGF und Analogen von KGF besteht.

44. Verwendung eines GLP-2-Rezeptor-Agonisten und wenigstens eines Peptidhormons bei der Herstellung von kultiviertem Gewebe des oberen Magen-Darm-Trakts, wie in Anspruch 43 gewonnen, zur Verwendung zum Wiederherstellen des oberen Magen-Darm-Trakts eines Patienten, bei der das Gewebe zum Implantieren in den Patienten bestimmt ist.

45. Verfahren zum Bestimmen der Aktivität eines Hormons, wenn es in Kombination mit GLP-2 verwendet wird, wobei das Verfahren die folgenden Schritte umfaßt:
(a) Verabreichen des Hormons zusammen mit einer Menge von GLP-2 oder eines GLP-2-Analogs an ein Versuchssäugetier,
(b) Abschätzen des anschließenden Wachstums von Gewebe des oberen Magen-Darm-Trakts bei dem Versuchssäugetier und
(c) Bestimmen, ob das Wachstum von Gewebe des oberen Magen-Darm-Trakts bei dem Versuchssäugetier im Verhältnis zu nur mit GLP-2 behandelten Kontrollsäugetieren gesteigert wird.

46. Verwendung von GLP-2 oder einem Analog von GLP-2 und wenigstens einem für eine Behandlung von Ulcus pepticum wirksamen anderen Wirkstoff bei der Herstellung eines Medikaments zum Behandeln eines Zielorganismus' mit Ulcus pepticum, bei der das Medikament dafür vorgesehen ist, an den oberen Magen-Darm-Trakt des Zielorganismus' abgegeben zu werden, und der wenigstens eine für eine Behandlung von Ulcus pepticum wirksame andere Wirkstoff in einer wirksamen therapeutischen Menge aus der Gruppe ausgewählt wird, die aus Wirkstoffen, die darauf wirken; die Säureausscheidung aus dem Magen zu blockieren, Wirkstoffen, die darauf wirken, eine schützende Sperre im Magen zu bilden, oder wismuthaltigen Verbindungen besteht.

47. Verwendung nach Anspruch 46, bei der die Wirkstoffe, die darauf wirken, die Säureausscheidung aus dem Magen zu blockieren, H₂-Rezeptor-Antagonisten, Protonenpumpenhemmer und Prostaglandine einschließen.

48. Verwendung nach Anspruch 46, bei der die Wirkstoffe, die darauf wirken, eine schützende Sperre im Magen zu bilden, Sucralfat einschließen.

49. Verwendung nach einem der Ansprüche 1 bis 12, 19 bis 38 und 43 bis 48, bei der das GLP-2 ein Wirbeltier-GLP-2 ist.

50. Verwendung nach einem der Ansprüche 1 bis 12, 19 bis 38 und 43 bis 48, bei der das GLP-2 ein Säugetier-GLP-2 ist.

51. Verwendung nach Anspruch 50, bei der das GLP-2 Human-GLP-2 ist.

52. Verwendung nach einem der Ansprüche 1 bis 12, 19 bis 38 und 43 bis 48, bei der das GLP-2 ein Analog von GLP-2 ist, das wenigstens einen Aminosäurezusatz, -austausch oder -verlust einschließt.

53. Verwendung nach Anspruch 52, bei der das GLP-2-Analog einen Aminosäureaustausch einschließt.

54. Verwendung nach Anspruch 53, bei der das GLP-2-Analog Human- oder Ratten-GLP-2-Analog ist und einen Aminosäureaustausch an Position 2 einschließt, der dem Analog eine Beständigkeit gegen ein Aufspalten durch das DPP-IV-Enzym verleiht.

55. Verwendung nach Anspruch 54, bei der das GLP-2-Analog Human-GLP-2-Analog ist und einen Glycinaustausch an Position 2 einschließt.

## Revendications

1. Utilisation d'un agoniste du récepteur GLP-2 dans la fabrication d'un médicament pour stimuler le fonctionnement du tractus gastro-intestinal supérieur, l'oesophage et l'estomac inclusivement, où le médicament est destiné à être fourni au tractus gastro-intestinal supérieur.

2. Utilisation selon la revendication 1, dans laquelle l'agoniste du récepteur GLP-2 est du GLP-2 ou un analogue peptidique de GLP-2, qui est destiné à être fourni à l'oesophage.

3. Utilisation selon la revendication 1, dans laquelle l'agoniste du récepteur GLP-2 est du GLP-2 ou un analogue peptidique de GLP-2, qui est destiné à être fourni à l'estomac.

4. Utilisation d'un analogue peptidique de GLP-2, dans la fabrication d'un médicament, pour faire proliférer le tissu dans le tractus gastro-intestinal supérieur, où le médicament est destiné à être fourni au tractus gastro-intestinal supérieur.

5. Utilisation selon les revendications 1 ou 4, où le sujet souffre d'une condition inflammatoire englobant le tractus gastro-intestinal supérieur.

6. Utilisation selon la revendication 5, où la condition inflammatoire englobant le tractus gastro-intestinal supérieur, est une condition inflammatoire englobant l'oesophage.

7. Utilisation selon la revendication 6, dans laquelle la condition inflammatoire englobant l'oesophage est sélectionnée parmi le groupe consistant en vascularite inflammatoire, inflammation post-infection, trouble d'infiltration, oesophagite infectieuse, oesophagite non infectieuse, sarcoïdose, maladie de Crohn, maladie de Behcet, réaction de greffon contre l'hôte, reflux acide, reflux biliaire, lésion due aux médicaments, lésion due aux produits chimiques, lésion due à l'irradiation, myosite ou collagénoses vasculaires.

8. Utilisation selon la revendication 5, où la condition inflammatoire englobant le tractus gastro-intestinal supérieur est une condition inflammatoire englobant l'estomac.

9. Utilisation selon la revendication 8, où la condition englobant l'estomac est sélectionnée parmi le groupe consistant en gastrite hémorragique et érosive, gastrite à *Helicobacter pylori*, gastrite chimique, gastrite atrophique métaplasique, gastrite atrophique post-antrectomie, gastrite éosinophile, gastrite infectieuse, maladie de Crohn, sarcoïdose, gastrite granulomateuse isolée, gastrite lymphocytaire et maladie de Ménétrier.

10. Utilisation selon la revendication 4, où le sujet a subi un résection partielle ou sous-totale du tractus gastro-intestinal supérieur.

11. Utilisation selon la revendication 10, où la résection partielle ou totale du tractus gastro-intestinal supérieur englobe l'oesophage ou l'estomac.

12. Utilisation selon les revendications 1-11, où le sujet est un humain.

13. Utilisation selon la revendication 12, où l'analogue de GLP-2 possède une activité stimulée de prolifération des cellules du tractus gastro-intestinal supérieur par rapport au GLP-2 natif de rat.

14. Utilisation selon la revendication 13, où l'analogue de GLP-2 est un analogue du GLP-2 humain.

15. Utilisation selon la revendication 14, où l'analogue de GLP-2 est destiné à être fourni au tractus gastro-intestinal supérieur par administration orale, sous-cutanée ou intraveineuse.

16. Méthode d'identification de peptides utiles dans le traitement de conditions inflammatoires englobant le tractus gastro-intestinal supérieur, comprenant les étapes consistant à:
a) obtenir un analogue d'un peptide GLP-2 de vertébrés, l'analogue ayant au moins une substitution, délétion, addition d'acide aminé, ou bien un acide aminé avec un groupe bloquant;
b) induire une condition inflammatoire englobant le tractus gastro-intestinal supérieur chez un animal d'expérimentation; et
c) déterminer l'effet de l'analogue sur l'état de santé ou la mortalité de l'animal d'expérimentation présentant une condition inflammatoire induite du tractus gastro-intestinal supérieur, comparé à des animaux témoins ne recevant pas le peptide, ou déterminer l'effet de l'analogue sur le poids du tractus gastro-intestinal supérieur des animaux d'expérimentation, comparés à des animaux témoins ne recevant pas le peptide.

17. Méthode d'identification de peptides utiles dans la prévention ou l'amélioration de conditions inflammatoires englobant le tractus gastro-intestinal supérieur, comprenant les étapes consistant à:
a) obtenir un analogue d'un peptide GLP-2 de vertébrés, l'analogue ayant au moins une substitution, délétion, addition d'acide aminé, ou bien un acide aminé avec un groupe bloquant;
b) induire une condition inflammatoire englobant le tractus gastro-intestinal supérieur chez un animal d'expérimentation présentant une condition inflammatoire du tractus gastro-intestinal supérieur comparé à des animaux témoins; et
c) déterminer l'effet de l'analogue sur l'état de santé ou la mortalité de l'animal d'expérimentation comparé à des animaux témoins ne recevant pas le peptide, ou déterminer l'effet de l'analogue sur le poids du tractus gastro-intestinal supérieur des animaux d'expérimentation, comparés à des animaux témoins ne recevant pas le peptide.

18. Méthode utile pour identifier des peptides capables de faire proliférer le tissu du tractus gastro-intestinal supérieur, comprenant les étapes consistant à:
a) obtenir un analogue d'un peptide GLP-2 de vertébrés, l'analogue ayant au moins une substitution, délétion, addition d'acide aminé, ou bien un acide aminé avec un groupe bloquant;
b) fournir l'analogue au tractus gastro-intestinal supérieur de l'animal d'expérimentation en utilisant un schéma capable de provoquer une prolifération tissulaire du tractus gastro-intestinal supérieur lorsqu'utzlisé pour le GLP-2 natif; et
c) évaluer l'augmentation dans la masse, la longueur ou la teneur totale en protéines du tractus gastro-intestinal supérieur ou d'une partie représentative, après l'achèvement du schéma de traitement.

19. Utilisation de GLP-2 ou d'un analogue de GLP-2 dans la fabrication d'un médicament pour inhiber l'apparition et/ou améliorer le développement d'une condition inflammatoire englobant l'inflammation du tractus gastro-intestinal supérieur chez un sujet à risque de développer une telle condition.

20. Utilisation d'un agoniste du récepteur GLP-2 et d'au moins une hormone peptidique dans la fabrication d'un médicament pour promouvoir la croissance tissulaire du tractus gastro-intestinal supérieur chez un mammifère, de telle sorte que les taux sériques dudit agoniste du récepteur et de ladite hormone peptidique sont élevés, et où l'agoniste du récepteur GLP-2 est du GLP-2 ou un analogue de GLP-2, et la au moins une autre hormone peptidique est sélectionnée parmi le groupe consistant en IGF-1, analogues de IGF-1, IGF-2, analogues de IGF-2, GH, analogues de GH, EGF, analogues de EGF, HGF, analogues de HGF, KGF, et analogues de KGF.

21. Utilisation selon la revendication 20, comprenant co-administrer à un mammifère, une quantité effective de GLP-2, ou d'un analogue de GLP-2, et une quantité effective d'au moins une autre hormone peptidique sélectionnée parmi le groupe consistant en IGF-1, analogues de IGF-1, IGF-2, analogues de IGF-2, GH, et analogues de GH.

22. Utilisation selon la revendication 20, où les cellules ont été produites par génie génétique *ex vivo*, afin de produire le GLP-2 et/ou l'autre hormone peptidique, et sont destinées à être implantées chez le mammifère,

23. Utilisation selon la revendication 20, où le GLP-2 et/ou l'autre hormone peptidique doivent être produits chez le mammifère par la production *in vivo* de cellules par génie génétique.

24. Utilisation selon la revendication 20, où l'hormone peptidique est de l'IGF-1 ou du LRIGF-1.

25. Utilisation selon la revendication 20, où l'hormone peptidique est de l'IGF-2.

26. Utilisation selon la revendication 20, où l'hormone peptidique est de la GH.

27. Utilisation selon la revendication 20 pour promouvoir la croissance de l'oesophage.

28. Utilisation selon la revendication 20 pour promouvoir la croissance de l'estomac.

29. Utilisation d'un agoniste du récepteur GLP-2 et d'au moins une hormone peptidique dans la fabrication d'un médicament pour stimuler le fonctionnement du tractus gastro-intestinal supérieur, de telle sorte que les taux sériques dudit agoniste du récepteur et de ladite hormone peptidique sont élevés, et où l'agoniste du récepteur GLP-2 est du GLP-2 ou un analogue de GLP-2, et la au moins une autre hormone peptidique est sélectionnée parmi le groupe consistant en IGF-1, analogues de IGF-1, IGF-2, analogues de IGF-2, GH, et analogues de GH, EGF, analogues d'EGF, HGF, analogues de HGF, KGF et analogues de KGF.

30. Utilisation selon la revendication 29 pour préserver, rétablir ou maintenir la fonction du tractus gastro-intestinal supérieur:
après un traitement de chimiothérapie ou de radiothérapie;
après une période d'alimentation parentérale;
après une maladie gastro-intestinale; ou
lorsque ledit patient est un enfant prématuré.

31. Utilisation selon la revendication 29, où le sujet souffre d'une condition inflammatoire englobant le tractus gastro-intestinal supérieur.

32. Utilisation selon la revendication 31, où la condition inflammatoire englobant le tractus gastro-intestinal supérieur est une condition inflammatoire englobant l'oesophage.

33. Utilisation selon la revendication 32, où la condition inflammatoire englobant l'oesophage est sélectionnée parmi le groupe consistant en vascularite inflammatoire, inflammation post-infection, trouble d'infiltration, oesophagite infectieuse, oesophagite non infectieuse, sarcoïdose, maladie de Crohn, maladie de Behcet, réaction de greffon contre l'hôte, reflux acide, reflux biliaire, lésion due aux médicaments, lésion due aux produits chimiques, lésion due à l'irradiation, myosite ou collagénoses vasculaires.

34. Utilisation selon la revendication 31, où la condition inflammatoire englobant le tractus gastro-intestinal supérieur est une condition inflammatoire englobant l'estomac.

35. Utilisation selon la revendication 34, où la condition inflammatoire englobant l'estomac est sélectionnée parmi le groupe consistant en gastrite hémorragique et érosive, gastrite à *Helicobacter pylori*, gastrite chimique, gastrite atrophique métaplasique, gastrite atrophique post-antrectomie, gastrite éosinophile, gastrite infectieuse, maladie de Crohn, sarcoïdose, gastrite granulomateuse isolée, gastrite lymphocytaire, ulcération de l'estomac et maladie de Ménétrier.

36. Utilisation selon la revendication 29, où le sujet a subi une résection partielle ou sous-totale du tractus gastro-intestinal supérieur.

37. Utilisation selon la revendication 36, où la résection partielle ou totale du tractus gastro-intestinal supérieur englobe l'oesophage ou l'estomac.

38. Utilisation selon les revendications 29-37, où le sujet est un humain.

39. Utilisation selon la revendication 38, où l'analogue de GLP-2 a une activité stimulée de prolifération des cellules du tractus gastro-intestinal supérieur par rapport au GLP-2 natif de rat.

40. Utilisation selon la revendication 39, où l'analogue de GLP-2 est un analogue du GLP-2 humain.

41. Utilisation selon la revendication 40, où l'analogue de GLP-2 est destiné à être fourni au tractus gastro-intestinal supérieur par administration orale, sous-cutanée ou intraveineuse.

42. Kit comprenant du GLP-2 ou un analogue de celui-ci et au moins une autre hormone peptidique sélectionnée parmi le groupe consistant en IGF-1, IGF-2, GH, EGF, HGF et KGF en une dose unitaire thérapeutiquement efficace ou en une quantité de dose multiple.

43. Méthode pour la promotion de la croissance du tissu ou des cellules du tractus gastro-intestinal supérieur, qui comprend l'étape consistant à cultiver ledit tissu ou lesdites cellules dans un milieu de culture contenant une combinaison promotrice de croissance de GLP-2, ou d'un analogue de GLP-2 et d'au moins une autre hormone peptidique sélectionnée parmi le groupe consistant en IGF-1, analogues d'IGF-1, IGF-2, analogues d'IGF-2, GH, analogues de GH, EGF, analogues d'EGF, HGF, analogues de HGF, KGF et analogues de KGF.

44. Utilisation d'un agoniste du récepteur GLP-2 et d'au moins une hormone peptidique pour la fabrication de tissu cultivé du tractus gastro-intestinal supérieur tel qu'obtenu dans la revendication 43, à utiliser pour rétablir le tractus gastro-intestinal supérieur d'un patient, où le tissu est destiné à être implanté chez le patient.

45. Méthode pour déterminer l'activité d'une hormone lorsqu'utilisée en conjonction avec du GLP-2, ladite méthode comprenant les étapes consistant à:
(a) co-administrer l'hormone avec une quantité de GLP-2 ou d'un analogue de GLP-2, à un animal d'expérimentation,
(b) évaluer la croissance subséquente du tissu du tractus gastro-intestinal supérieur chez l'animal d'expérimentation, et
(c) déterminer si la croissance du tissu du tractus gastro-intestinal supérieur chez l'animal d'expérimentation est stimulée par rapport aux mammifères témoins traités avec du GLP-2 seul.

46. Utilisation de GLP-2 ou d'un analogue de GLP-2, et d'au moins un autre agent efficace pour traiter des ulcères gastroduodénaux, dans la fabrication d'un médicament pour traiter un sujet ayant des ulcères gastroduodénaux, où le médicament est destiné à être fourni au tractus gastro-intestinal supérieur du sujet, et le au moins un autre agent efficace pour traiter des ulcères gastroduodénaux est sélectionné parmi le groupe consistant en: agents qui agissent pour bloquer la sécrétion d'acide de l'estomac, agents qui agissent pour former une barrière protectrice dans l'estomac, ou des composés contenant du bismuth, en une quantité thérapeutiquement effective.

47. Utilisation selon la revendication 46, dans laquelle les agents qui agissent pour bloquer la sécrétion d'acide de l'estomac, comprennent des antagonistes du récepteur H₂, des inhibiteurs de la pompe à protons et des prostaglandines.

48. Utilisation selon la revendication 46, où les agents qui agissent pour former une barrière protectrice dans l'estomac, comprennent du sucralfate.

49. Utilisation selon l'une quelconque des revendications 1-12, 19-38 et 43-48, où le GLP-2 est un GLP-2 de vertébrés.

50. Utilisation selon l'une quelconque des revendications 1-12, 19-38 et 43-48, où le GLP-2 est un GLP-2 mammalien.

51. Utilisation selon la revendication 50, où le GLP-2 est du GLP-2 humain.

52. Utilisation selon l'une quelconque des revendications 1-12, 19-38 et 43-48, où le GLP-2 est un analogue de GLP-2 qui incorpore au moins une addition, substitution ou délétion d'acide aminé.

53. Utilisation selon la revendication 52, où l'analogue de GLP-2 incorpore une substitution d'acide aminé,

54. Utilisation selon la revendication 53, où l'analogue de GLP-2 est un analogue de GLP-2 humain ou de rat et incorpore une substitution d'acide aminé en position 2, laquelle confère audit analogue une résistance au clivage par l'enzyme DPP-IV.

55. Utilisation selon la revendication 54, où l'analogue de GLP-2 est un analogue de GLP-2 humain et incorpore une substitution glycine en position 2.
